# EUROPEAN PATENT SPECIFICATION

(11) **EP 3 561 069 B1**
(45) Date of publication and mention of the grant of the patent: **24.07.2024**
(21) Application number: 17888390.6
(22) Date of filing: 21.12.2017
(51) Int. Cl.: C12Q 1/68, C12Q 1/6869, C12Q 1/6886, C12Q 1/689

(54) **METHOD FOR DIAGNOSING LUNG CANCER VIA BACTERIAL METAGENOMIC ANALYSIS**
VERFAHREN ZUR DIAGNOSE VON LUNGENKREBS DURCH BAKTERIELLE METAGENOME ANALYSE
PROCÉDÉ DE DIAGNOSTIC DU CANCER DU POUMON PAR ANALYSE MÉTAGÉNOMIQUE BACTÉRIENNE

(30) Priority: 26.12.2016 KR 20160179225; 27.06.2017 KR 20170080905; 27.06.2017 KR 20170080906
(43) Date of publication of application: 30.10.2019
(73) Proprietor: MD Healthcare Inc., Seoul 03923 (KR)
(72) Inventor: KIM, Yoon-Keun, Gyeonggi-do 10908 (KR)
(74) Representative: Dehns
(86) International application number: PCT/KR2017/015173
(87) International publication number: WO 2018/124617

(56) References cited:
- EP-A2- 2 484 752
- WO-A1-2016/133324
- KR-A- 20110 025 603
- KR-A- 20120 114 872
- KR-A- 20130 021 920
- KR-A- 20160 101 521
- YOU-SUN KIM ET AL: "IgG Sensitization to Extracellular Vesicles in Indoor Dust Is Closely Associated With the Prevalence of Non-Eosinophilic Asthma, COPD, and Lung Cancer", ALLERGY, ASTHMA & IMMUNOLOGY RESEARCH : AAIR, vol. 8, no. 3, 1 May 2016 (2016-05-01), US, pages 198, XP055713012, ISSN: 2092-7355, DOI: 10.4168/aair.2016.8.3.198
- DAYLE JOHNSTON ET AL: "Illumina MiSeq 16S amplicon sequence analysis of bovine respiratory disease associated bacteria in lung and mediastinal lymph node tissue", BMC VETERINARY RESEARCH, vol. 13, no. 1, 2 May 2017 (2017-05-02), XP055713124, DOI: 10.1186/s12917-017-1035-2
- JINHO YANG ET AL: "Lung Disease Diagnostic Model Through IgG Sensitization to Microbial Extracellular Vesicles", ALLERGY, ASTHMA & IMMUNOLOGY RESEARCH : AAIR, vol. 12, no. 4, 1 July 2020 (2020-07-01), US, pages 669 - 683, XP055713008, ISSN: 2092-7355, DOI: 10.4168/aair.2020.12.4.669
- YAN, X. ET AL.: "Discovery and Validation of Potential Bacterial Biomarkers for Lung Cancer", AMERICAN JOURNAL OF CANCER RESEARCH, vol. 5, no. 10, 15 September 2015 (2015-09-15), pages 3111 - 3122, XP055516990

## Description

### [Technical Field]

The present invention relates to a method of diagnosing lung cancer through bacterial metagenomic analysis, and more particularly, to a method of diagnosing lung cancer by analyzing an increase or decrease in content of extracellular vesicles derived from specific bacteria by bacterial metagenomic analysis using a subject-derived sample.

### [Background Art]

Lung cancer is a malignant tumor originating from the lungs, and, in spite of the development of modern medical science, lung cancer has a 5-year survival rate of less than 50% and is the leading cause of death in Korea. Lung cancer is broadly classified into small cell lung cancer and non-small cell lung cancer according to histological type, and the small cell lung cancer has a feature distinctively distinguished from other types of lung cancer in terms of treatment and prognosis, and thus histological examination results of lung cancer are very important in determining a decision for therapeutic policy.

Lung cancer has symptoms such as coughing, hemoptysis, chest pain, dyspnea, and the like. However, when such symptoms are shown, there are many cases where lung cancer has already progressed, and frequent cases where there are no symptoms yet lung cancer is progressing, and only 5% to 15% of lung cancer patients are diagnosed when they have no symptoms and most lung cancer patients are diagnosed with lung cancer after experiencing such symptoms. So far, there has been no certified screening method for early detection of lung cancer before symptoms are shown, early detection of lung cancer using chest computed tomography (CT) is most commonly used, but the effectiveness thereof has yet been proved. Thus, there is an urgent need to develop a method of enhancing therapeutic efficiency by early detection of lung cancer, and, prior to the development of such method, it is very important to differentiate methods for early diagnosis and treatment by predicting the risk for lung cancer, and thus research into and development of these methods are required.

Meanwhile, it is known that the number of microorganisms symbiotically living in the human body is 100 trillion which is 10 times the number of human cells, and the number of genes of microorganisms exceeds 100 times the number of human genes. A microbiota or microbiome is a microbial community that includes bacteria, archaea, and eukaryotes present in a given habitat, and intestinal microbiota is known to play a vital role in human's physiological phenomena and significantly affect human health and diseases through interactions with human cells. Bacteria symbiotically living in the human body secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacterial-derived vesicles have a size of approximately 100 nm or less and thus relatively freely pass through the mucous membranes and are absorbed into the human body.

With regard to the onset of lung cancer, chronic obstructive pulmonary disease (COPD) is a vital risk factor, and in clinical studies, it has been reported that COPD itself is a vital risk factor for the onset of lung cancer regardless of smoking. In addition, the fact that the onset of lung cancer has recently increased in non-smokers suggests that there is a causative factor for the onset of lung cancer other than smoking.

Metagenomics, also called environmental genomics, may be analytics for metagenomic data obtained from samples collected from the environment, and collectively refers to a total genome of all microbiota in the natural environment in which microorganisms exist and was first used by Jo Handelsman in 1998 (Handelsman et al., 1998 Chem. Biol. 5, R245-249). Recently, the bacterial composition of human microbiota has been listed using a method based on 16s ribosomal RNA (16s rRNA) base sequences, and 16s ribosomal RNA analyzes sequences using a 454FLX titanium platform. EP2484752 A2 provides a method for the diagnosis of diseases caused by Gram positive bacteria-derived extracellular vesicles wherein the method comprises measuring 16S rRNA by PCR.

In the onset of lung cancer, however, identification of causative factors of lung cancer through metagenomic analysis of bacteria-derived vesicles isolated from a human-derived substance, such as blood or the like, and a method of predicting lung cancer have never been reported.

### [Disclosure]

### [Technical Problem]

To diagnose lung cancer based on causative factors thereof, the inventors of the present invention extracted DNA from bacteria-derived extracellular vesicles isolated from blood, which is a subject-derived sample, and performed metagenomic analysis on the extracted DNA, and, as a result, identified bacteria-derived extracellular vesicles capable of acting as a causative factor of lung cancer, thus completing the present invention.

Therefore, it is an object of the present invention to provide a method of providing information for lung cancer diagnosis by metagenomic analysis of bacteria-derived extracellular vesicles.

However, the technical goals of the present invention are not limited to the aforementioned goals, and other unmentioned technical goals will be clearly understood by those of ordinary skill in the art from the following description.

### [Technical Solution]

According to an aspect of the present invention, there is provided a method of providing information for lung cancer diagnosis, comprising the following processes:
(a) extracting DNA from extracellular vesicles isolated from a subject sample;
(b) performing polymerase chain reaction (PCR) on the extracted DNA using a pair of primers having SEQ ID NO:1 and SEQ ID NO: 2; and
(c) making said diagnosis by, through sequencing of the PCR product, comparing an increase or decrease in content of bacteria-derived extracellular vesicles of the subject sample with that of a normal individual-derived sample, a chronic obstructive pulmonary disease patient-derived sample, or an asthma patient-derived sample through sequencing of a product of the PCR.

The present invention also provides a method of diagnosing lung cancer, comprising the following processes:
(a) extracting DNA from extracellular vesicles isolated from a subject sample;
(b) performing polymerase chain reaction (PCR) on the extracted DNA using a pair of primers having SEQ ID NO:1 and SEQ ID NO: 2; and
(c) making said diagnosis by, through sequencing of the PCR product, comparing an increase or decrease in content of bacteria-derived extracellular vesicles of the subject sample with that of a normal individual-derived sample, a chronic obstructive pulmonary disease patient-derived sample, or an asthma patient-derived sample through sequencing of a product of the PCR.

Also disclosed but not part of the invention, is a method of predicting a risk for lung cancer, comprising the following processes:
(a) extracting DNA from extracellular vesicles isolated from a subject sample;
(b) performing polymerase chain reaction (PCR) on the extracted DNA using a pair of primers having SEQ ID NO:1 and SEQ ID NO: 2; and
(c) comparing an increase or decrease in content of bacteria-derived extracellular vesicles of the subject sample with that of a normal individual-derived sample, a chronic obstructive pulmonary disease patient-derived sample, or an asthma patient-derived sample through sequencing of a product of the PCR.

In one embodiment of the present invention, in process (c), lung cancer may be diagnosed by comparing an increase or decrease in content of extracellular vesicles derived from one or more bacteria selected from the group consisting of the phylum *Acidobacteria* and the phylum *Chloroflexi* with that of the normal individual-derived sample.

In another embodiment of the present invention, in process (c), lung cancer may be diagnosed by comparing an increase or decrease in content of extracellular vesicles derived from one or more bacteria selected from the group consisting of the class *Thermomicrobia* and the class *Solibacteres* with that of the normal individual-derived sample.

In another embodiment of the present invention, in process (c), lung cancer may be diagnosed by comparing an increase or decrease in content of extracellular vesicles derived from one or more bacteria selected from the group consisting of the order *Turicibacterales,* the order *Rickettsiales,* the order *Alteromonadales,* the order RF32, the order *Thermales,* the order JG30-KF-CM45, the order I025, the order *Solibacterales,* and the order *Aeromonadales* with that of the normal individual-derived sample.

In another embodiment of the present invention, in process (c), lung cancer may be diagnosed by comparing an increase or decrease in content of extracellular vesicles derived from one or more bacteria selected from the group consisting of the family *Turicibacteraceae,* the family *Clostridiaceae,* the family S24-7, the family *Rhizobiaceae,* the family *mitochondria,* the family F16, the family *Gordoniaceae,* the family *Rhodospirillaceae,* the family *Thermaceae,* the family *Shewanellaceae,* the family Ellin6075, the family Rs-045, and the family *Aeromonadaceae* with that of the normal individual-derived sample.

In another embodiment of the present invention, in process (c), lung cancer may be diagnosed by comparing an increase or decrease in content of extracellular vesicles derived from one or more bacteria selected from the group consisting of the genus *Chromohalobacter,* the genus *Geobacillus,* the genus *Proteus,* the genus *Megamonas,* the genus *Moraxella,* the genus *Alloiococcus,* the genus *Turicibacter,* the genus SMB53, the genus *Veillonella,* the genus *Peptoniphilus,* the genus *Comamonas,* the genus *Hymenobacter,* the genus *Citrobacter,* the genus *Novosphingobium,* the genus *Gordonia,* the genus *Aerococcus,* the genus *Thermus,* the genus *Shewanella,* and the genus *Achromobacter* with that of the normal individual-derived sample.

In another embodiment of the present invention, in process (c), lung cancer may be diagnosed by comparing an increase or decrease in content of extracellular vesicles derived from one or more bacteria selected from the group consisting of the order *Bacillales,* the order *Rickettsiales,* and the order I025 with that of the chronic obstructive pulmonary disease patient-derived sample.

In another embodiment of the present invention, in process (c), lung cancer may be diagnosed by comparing an increase or decrease in content of extracellular vesicles derived from one or more bacteria selected from the group consisting of the family *Staphylococcaceae,* the family *Nocardiaceae,* and the family Rs-045 with that of the chronic obstructive pulmonary disease patient-derived sample.

In another embodiment of the present invention, in process (c), lung cancer may be diagnosed by comparing an increase or decrease in content of extracellular vesicles derived from one or more bacteria selected from the group consisting of the genus *Alloiococcus,* the genus *Moraxella,* the genus *Staphylococcus,* the genus *Brevundimonas,* the genus *Enhydrobacter,* the genus *Comamonas,* and the genus *Rhodococcus* with that of the chronic obstructive pulmonary disease patient-derived sample.

In another embodiment of the present invention, in process (c), lung cancer may be diagnosed by comparing an increase or decrease in content of extracellular vesicles derived from one or more bacteria selected from the group consisting of the phylum *Bacteroidetes,* the phylum *Cyanobacteria,* the phylum TM7, the phylum *Fusobacteria,* the phylum *Thermi,* the phylum *Verrucomicrobia,* the phylum *Armatimonadetes,* the phylum *Acidobacteria,* the phylum *Gemmatimonadetes,* and the phylum *Chloroflexi* with that of the asthma patient-derived sample.

In another embodiment of the present invention, in process (c), lung cancer may be diagnosed by comparing an increase or decrease in content of extracellular vesicles derived from one or more bacteria selected from the group consisting of the class *Bacteroidia,* the class *Bacilli,* the class *Flavobacteriia,* the class *Sphingobacteriia,* the class *Alphaproteobacteria,* the class *Fusobacteriia,* the class TM7-3, the class *Deinococci,* the class *Verrucomicrobiae,* the class *Saprospirae,* the class *Chloroplast,* the class *Cytophagia,* the class *Fimbriimonadia,* the class *Chloracidobacteria,* the class *Thermomicrobia,* the class *Thermoleophilia,* and the class *Solibacteres* with that of the asthma patient-derived sample.

In another embodiment of the present invention, in process (c), lung cancer may be diagnosed by comparing an increase or decrease in content of extracellular vesicles derived from one or more bacteria selected from the group consisting of the order YS2, the order *Turicibacterales,* the order *Bifidobacteriales,* the order *Bacteroidales,* the order *Enterobacteriales,* the order *Rhodobacterales,* the order *Gemellales,* the order *Flavobacteriales,* the order *Caulobacterales,* the order *Neisseriales,* the order *Sphingobacteriales,* the order *Deinococcales,* the order *Pseudomonadales,* the order *Rhodocyclales,* the order *Xanthomonadales,* the order *Fusobacteriales,* the order *Actinomycetales,* the order *Sphingomonadales,* the order *Verrucomicrobiales,* the order *Saprospirales,* the order *Rhizobiales,* the order *Bacillales,* the order *Streptophyta,* the order *Cytophagales,* the order *Thermales,* the order *Fimbriimonadales,* the order CW040, the order *Rickettsiales,* the order RB41, the order *Alteromonadales,* the order JG30-KF-CM45, the order I025, the order *Aeromonadales,* and the order *Solibacterales* with that of the asthma patient-derived sample.

In another embodiment of the present invention, in process (c), lung cancer may be diagnosed by comparing an increase or decrease in content of extracellular vesicles derived from one or more bacteria selected from the group consisting of the family *Helicobacteraceae,* the family *Bacteroidaceae,* the family *Turicibacteraceae,* the family *Veillonellaceae,* the family *Bijidobacteriaceae,* the family *Barnesiellaceae,* the family *Rikenellaceae,* the family *Clostridiaceae,* the family *Odoribacteraceae,* the family *Enterobacteriaceae,* the family *Porphyromonadaceae,* the family *Gemellaceae,* the family *Weeksellaceae,* the family *Carnobacteriaceae,* the family *Leptotrichiaceae,* the family *Moraxellaceae,* the family *Caulobacteraceae,* the family *Erythrobacteraceae,* the family *Hyphomicrobiaceae,* the family *Neisseriaceae,* the family *Sphingobacteriaceae,* the family *Deinococcaceae,* the family *Aerococcaceae,* the family *Bartonellaceae,* the family *Micrococcaceae,* the family *Flavobacteriaceae,* the family *Burkholderiaceae,* the family *Lactobacillaceae,* the family *Dietziaceae,* the family *Rhodocyclaceae,* the family *Xanthomonadaceae,* the family *Geodermatophilaceae,* the family *Actinomycetaceae,* the family *Methylobacteriaceae,* the family *Pseudomonadaceae,* the family *Corynebacteriaceae,* the family *Staphylococcaceae,* the family *Nocardioidaceae,* the family *Verrucomicrobiaceae,* the family *Sphingomonadaceae,* the family *Mycobacteriaceae,* the family *Tissierellaceae,* the family *Chitinophagaceae,* the family *Intrasporangiaceae,* the family *Propionibacteriaceae,* the family *Aurantimonadaceae,* the family *Planococcaceae,* the family *Fusobacteriaceae,* the family *Bradyrhizobiaceae,* the family *Nocardiaceae,* the family *Dermabacteraceae,* the family *Bacillaceae,* the family *Thermaceae,* the family Ellin6075, the family *Brevibacteriaceae,* the family *Microbacteriaceae,* the family *Rhodospirillaceae,* the family *Cytophagaceae,* the family *Fimbriimonadaceae,* the family *Dermacoccaceae,* the family *Chromatiaceae,* the family *Rhizobiaceae,* the family *Gordonžaceae,* the family *mitochondria,* the family *Pseudonocardiaceae,* the family *Exiguobacteraceae,* the family *Shewanellaceae,* the family F16, the family Rs-045, and the family *Aeromonadaceae* with that of the asthma patient-derived sample.

In another embodiment of the present invention, in process (c), lung cancer may be diagnosed by comparing an increase or decrease in content of extracellular vesicles derived from one or more bacteria selected from the group consisting of the genus *Trabulsiella,* the genus *Enterobacter,* the genus *Veillonella,* the genus *Bifidobacterium,* the genus *Lachnospira,* the genus *Comamonas,* the genus *Bacteroides,* the genus *Turicibacter,* the genus *Sutterella,* the genus *Klebsiella,* the genus SMB53, the genus *Roseburia,* the genus *Dialister,* the genus *Ruminococcus,* the genus *Parabacteroides,* the genus *Butyricimonas,* the genus *Odoribacter,* the genus *Eubacterium,* the genus *Dorea,* the genus *Enhydrobacter,* the genus *Granulicatella,* the genus *Chryseobacterium,* the genus *Porphyromonas,* the genus *Coprococcus,* the genus *Peptoniphilus,* the genus *Microbispora,* the genus *Deinococcus,* the genus *Acinetobacter,* the genus *Aerococcus,* the genus *Actinomyces,* the genus *Brevundimonas,* the genus *Blastomonas,* the genus *Citrobacter,* the genus *Lactobacillus,* the genus *Stenotrophomonas,* the genus *Corynebacterium,* the genus *Pseudomonas,* the genus *Lautropia,* the genus *Akkermansia,* the genus *Staphylococcus,* the genus *Bacillus,* the genus *Sphingobacterium,* the genus *Anaerococcus,* the genus *Neisseria,* the genus *Leptotrichia,* the genus *Mycobacterium,* the genus *Kocuria,* the genus *Methylobacterium,* the genus *Propionibacterium,* the genus *Hymenobacter,* the genus *Sphingomonas,* the genus *Fusobacterium,* the genus *Brachybacterium,* the genus *Rhodococcus,* the genus *Micrococcus,* the genus *Kaistobacter,* the genus *Finegoldia,* the genus *Rubellimicrobium,* the genus *Brevibacterium,* the genus *Agrobacterium,* the genus *Dietzia,* the genus *Fimbriimonas,* the genus *Flavobacterium,* the genus *Dermacoccus,* the genus *Skermanella,* the genus *Novosphingobium,* the genus *Gordonia,* the genus *Rheinheimera,* the genus *Achromobacter,* the genus *Hydrogenophilus,* the genus *Thermus,* the genus *Exiguobacterium,* the genus *Shewanella,* the genus *Ralstonia,* and the genus *Alkanindiges* with that of the asthma patient-derived sample.

In another embodiment of the present invention, the subject sample may be blood.

In another embodiment of the present invention, the blood may be whole blood, serum, plasma, or blood mononuclear cells.

### [Advantageous Effects]

Extracellular vesicles secreted from bacteria present in the environment are absorbed into the human body, and thus can directly affect the occurrence of cancer, and since it is difficult to implement early diagnosis for lung cancer before symptoms are shown, efficient treatment is difficult. Thus, according to the present invention, a risk for lung cancer may be predicted through metagenomic analysis of bacteria or bacteria-derived extracellular vesicles using a human body-derived sample, and thus the onset of lung cancer can be delayed or lung cancer can be prevented through appropriate management by early diagnosis and prediction of a risk group for lung cancer, and, even after lung cancer occurs, early diagnosis for lung cancer can be implemented, thereby lowering a disease rate and increasing therapeutic effects. In addition, causative factors can be predicted by performing metagenomic analysis on patients diagnosed with lung cancer, and thus the patients are able to avoid exposure to the causative factors, whereby the progression of lung cancer is ameliorated, or recurrence of lung cancer can be prevented.

### [Description of Drawings]

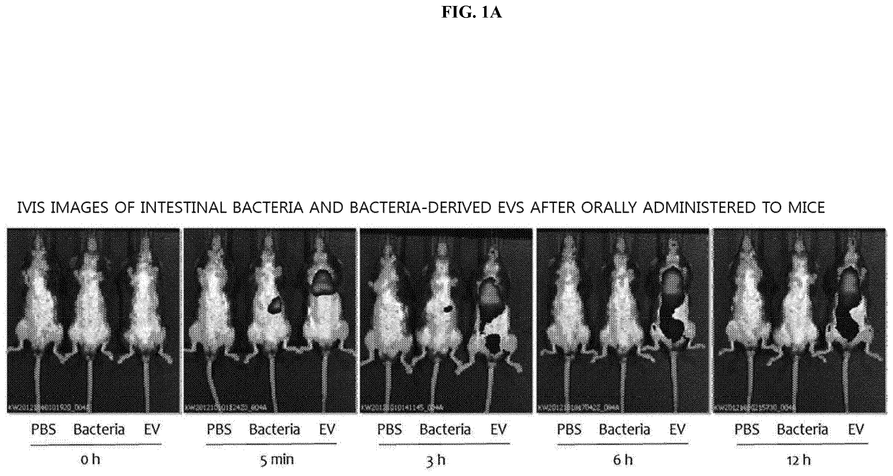

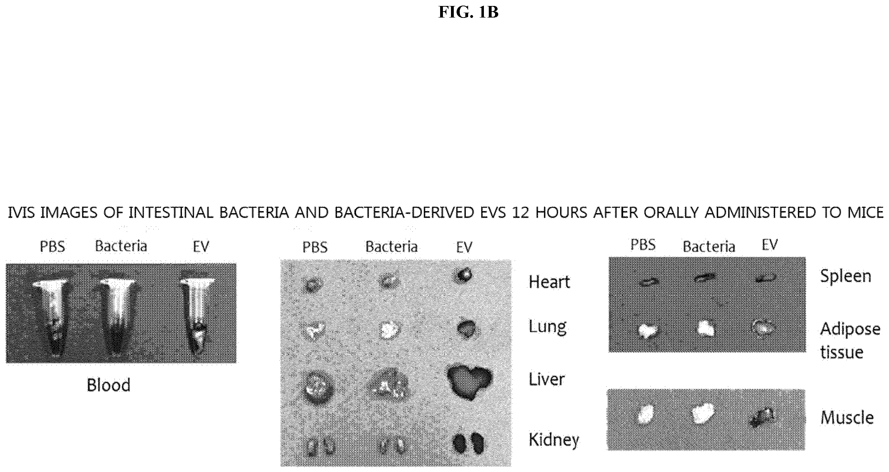

FIGS. 1A and 1B are views for evaluating the distribution pattern of extracellular vesicles derived from bacteria *in vivo.* FIG. 1A illustrates images showing the distribution pattern of intestinal bacteria and extracellular vehicles (EVs) derived from bacteria per time (0 h, 5 min, 3 h, 6 h, and 12 h) after being orally administered to mice. FIG. 1B illustrates images showing the distribution pattern of intestinal bacteria and EVs derived from bacteria after being orally administered to mice and, after 12 hours, blood and various organs (heart, lung, liver, kidney, spleen, adipose tissue, and muscle) of the mice were extracted.
FIG. 2 illustrates distribution results of bacteria-derived EVs exhibiting significant diagnostic performance at a phylum level, after metagenomic analysis of bacteria-derived EVs isolated from lung cancer patient-derived blood and normal individual-derived blood.
FIG. 3 illustrates distribution results of bacteria-derived EVs exhibiting significant diagnostic performance at a class level, after metagenomic analysis of bacteria-derived EVs isolated from lung cancer patient-derived blood and normal individual-derived blood.
FIG. 4 illustrates distribution results of bacteria-derived EVs exhibiting significant diagnostic performance at an order level, after metagenomic analysis of bacteria-derived EVs isolated from lung cancer patient-derived blood and normal individual-derived blood.
FIG. 5 illustrates distribution results of bacteria-derived EVs exhibiting significant diagnostic performance at a family level, after metagenomic analysis of bacteria-derived EVs isolated from lung cancer patient-derived blood and normal individual-derived blood.
FIG. 6 illustrates distribution results of bacteria-derived EVs exhibiting significant diagnostic performance at a genus level, after metagenomic analysis of bacteria-derived EVs isolated from lung cancer patient-derived blood and normal individual-derived blood.
FIG. 7 illustrates distribution results of bacteria-derived EVs exhibiting a significant diagnostic performance at an order level, after metagenomic analysis of bacteria-derived EVs isolated from lung cancer patient-derived blood and COPD patient-derived blood.
FIG. 8 illustrates distribution results of bacteria-derived EVs exhibiting significant diagnostic performance at a family level, after metagenomic analysis of bacteria-derived EVs isolated from lung cancer patient-derived blood and COPD patient-derived blood.
FIG. 9 illustrates distribution results of bacteria-derived EVs exhibiting significant diagnostic performance at a genus level, after metagenomic analysis of bacteria-derived EVs isolated from lung cancer patient-derived blood and COPD patient-derived blood.
FIG. 10 illustrates distribution results of bacteria-derived EVs exhibiting significant diagnostic performance at a phylum level, after metagenomic analysis of bacteria-derived EVs isolated from lung cancer patient-derived blood and asthma patient-derived blood.
FIG. 11 illustrates distribution results of bacteria-derived EVs exhibiting significant diagnostic performance at a class level, after metagenomic analysis of bacteria-derived EVs isolated from lung cancer patient-derived blood and asthma patient-derived blood.
FIG. 12 illustrates distribution results of bacteria-derived EVs exhibiting significant diagnostic performance at an order level, after metagenomic analysis of bacteria-derived EVs isolated from lung cancer patient-derived blood and asthma patient-derived blood.
FIG. 13 illustrates distribution results of bacteria-derived EVs exhibiting significant diagnostic performance at a family level, after metagenomic analysis of bacteria-derived EVs isolated from lung cancer patient-derived blood and asthma patient-derived blood.
FIG. 14 illustrates distribution results of bacteria-derived EVs exhibiting significant diagnostic performance at a genus level, after metagenomic analysis of bacteria-derived EVs isolated from lung cancer patient-derived blood and asthma patient-derived blood.

### [Mode of the Invention]

The present invention relates to a method of diagnosing lung cancer through bacterial metagenomic analysis. The inventors of the present invention extracted genes from bacteria-derived extracellular vesicles of a subject-derived sample, performed metagenomic analysis thereon, and identified bacteria-derived extracellular vesicles capable of acting as a causative factor of lung cancer.

Thus, the present invention provides a method of providing information for lung cancer diagnosis, the method comprising:
(a) extracting DNA from extracellular vesicles isolated from a subject sample;
(b) performing polymerase chain reaction (PCR) on the extracted DNA using a pair of primers having SEQ ID NOS: 1 and 2; and
(c) making said diagnosis by, through sequencing of the PCR product, comparing an increase or decrease in content of bacteria-derived extracellular vesicles of the subject sample with that of a normal individual-derived sample, a chronic obstructive pulmonary disease patient-derived sample, or an asthma patient-derived sample through sequencing of a product of the PCR.

The term "lung cancer diagnosis" as used herein refers to determining whether a patient has a risk for lung cancer, whether the risk for lung cancer is relatively high, or whether lung cancer has already occurred. The method of the present invention may be used to delay the onset of lung cancer through special and appropriate care for a specific patient, which is a patient having a high risk for lung cancer or prevent the onset of lung cancer. In addition, the method may be clinically used to determine treatment by selecting the most appropriate treatment method through early diagnosis of lung cancer.

The lung cancer diagnosis of the present invention is advantageous in that various subtypes of lung cancer, such as adenocarcinoma, small cell carcinoma, and squamous cell carcinoma can be simultaneously predicted.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, metagenomic analysis is performed using bacteria-derived extracellular vesicles isolated from serum.

In the present invention, the subject sample may be blood, and the blood may be whole blood, serum, plasma, or blood mononuclear cells, but the present invention is not limited thereto.

In an embodiment of the present invention, metagenomic analysis was performed on bacteria-derived extracellular vesicles in normal individual-derived blood and lung cancer patient-derived blood, and bacteria-derived extracellular vesicles capable of acting as a cause of the onset of lung cancer were actually identified by analysis at phylum, class, order, family, and genus levels.

More particularly, in one embodiment of the present invention, as a result of performing metagenomic analysis on bacteria-derived extracellular vesicles at a phylum level, the content of extracellular vesicles derived from bacteria belonging to the phylum *Acidobacteria* and the phylum *Chloroflexi* was significantly different between lung cancer patients and normal individuals (see Example 4).

More particularly, in one embodiment of the present invention, as a result of performing metagenomic analysis on bacteria-derived extracellular vesicles at a class level, the content of extracellular vesicles derived from bacteria belonging to the class *Thermomicrobia* and the class *Solibacteres* was significantly different between lung cancer patients and normal individuals (see Example 4).

More particularly, in one embodiment of the present invention, as a result of performing metagenomic analysis on bacteria-derived extracellular vesicles at an order level, the content of extracellular vesicles derived from bacteria belonging to the order *Turicibacterales,* the order *Rickettsiales,* the order *Alteromonadales,* the order RF32, the order *Thermales,* the order JG30-KF-CM45, the order I025, the order *Solibacterales,* and the order *Aeromonadales* was significantly different between lung cancer patients and normal individuals (see Example 4).

More particularly, in one embodiment of the present invention, as a result of performing metagenomic analysis on bacteria-derived extracellular vesicles at a family level, the content of extracellular vesicles derived from bacteria belonging to the family *Turicibacteraceae,* the family *Clostridiaceae,* the family S24-7, the family *Rhizobiaceae,* the family *mitochondria,* the family F16, the family *Gordoniaceae,* the family *Rhodospirillaceae,* the family *Thermaceae,* the family *Shewanellaceae,* the family Ellin6075, the family Rs-045, and the family *Aeromonadaceae* was significantly different between lung cancer patients and normal individuals (see Example 4).

More particularly, in one embodiment of the present invention, as a result of performing metagenomic analysis on bacteria-derived extracellular vesicles at a genus level, the content of extracellular vesicles derived from bacteria belonging to the genus *Chromohalobacter,* the genus *Geobacillus,* the genus *Proteus,* the genus *Megamonas,* the genus *Moraxella,* the genus *Alloiococcus,* the genus *Turicibacter,* the genus SMB53, the genus *Veillonella,* the genus *Peptoniphilus,* the genus *Comamonas,* the genus *Hymenobacter,* the genus *Citrobacter,* the genus *Novosphingobium,* the genus *Gordonia,* the genus *Aerococcus,* the genus *Thermus,* the genus *Shewanella,* and the genus *Achromobacter* was significantly different between lung cancer patients and normal individuals (see Example 4).

In another embodiment of the present invention, metagenomic analysis was performed on bacteria-derived extracellular vesicles in chronic obstructive pulmonary disease (COPD) patient-derived blood and lung cancer patient-derived blood, and bacteria-derived extracellular vesicles capable of acting as a cause of the onset of lung cancer were actually identified by analysis at phylum, class, order, family, and genus levels.

More particularly, in one embodiment of the present invention, as a result of performing metagenomic analysis on bacteria-derived extracellular vesicles at an order level, the content of extracellular vesicles derived from bacteria belonging to the order *Bacillales,* the order *Rickettsiales,* and the order I025 was significantly different between lung cancer patients and COPD patients (see Example 5).

More particularly, in one embodiment of the present invention, as a result of performing metagenomic analysis on bacteria-derived extracellular vesicles at a family level, the content of extracellular vesicles derived from bacteria belonging to the family *Staphylococcaceae,* the family *Nocardiaceae,* and the family Rs-045 was significantly different between lung cancer patients and COPD patients (see Example 5).

More particularly, in one embodiment of the present invention, as a result of performing metagenomic analysis on bacteria-derived extracellular vesicles at a genus level, the content of extracellular vesicles derived from bacteria belonging to the genus *Alloiococcus,* the genus *Moraxella,* the genus *Staphylococcus,* the genus *Brevundimonas,* the genus *Enhydrobacter,* the genus *Comamonas,* and the genus *Rhodococcus* was significantly different between lung cancer patients and COPD patients (see Example 5).

In another embodiment of the present invention, metagenomic analysis was performed on bacteria-derived extracellular vesicles in asthma patient-derived blood and lung cancer patient-derived blood, and bacteria-derived extracellular vesicles capable of acting as a cause of the onset of lung cancer were actually identified by analysis at phylum, class, order, family, and genus levels.

More particularly, in one embodiment of the present invention, as a result of performing metagenomic analysis on bacteria-derived extracellular vesicles at a phylum level, the content of extracellular vesicles derived from bacteria belonging to the phylum *Bacteroidetes,* the phylum *Cyanobacteria,* the phylum TM7, the phylum *Fusobacteria,* the phylum *Thermi,* the phylum *Verrucomicrobia,* the phylum *Armatimonadetes,* the phylum *Acidobacteria,* the phylum *Gemmatimonadetes,* and the phylum *Chloroflexi* was significantly different between lung cancer patients and asthma patients (see Example 6).

More particularly, in one embodiment of the present invention, as a result of performing metagenomic analysis on bacteria-derived extracellular vesicles at a class level, the content of extracellular vesicles derived from bacteria belonging to the class *Bacteroidia,* the class *Bacilli,* the class *Flavobacteriia,* the class *Sphingobacteriia,* the class *Alphaproteobacteria,* the class *Fusobacteriia,* the class TM7-3, the class *Deinococci,* the class *Verrucomicrobiae,* the class *Saprospirae,* the class *Chloroplast,* the class *Cytophagia,* the class *Fimbriimonadia,* the class *Chloracidobacteria,* the class *Thermomicrobia,* the class *Thermoleophilia,* and the class *Solibacteres* was significantly different between lung cancer patients and asthma patients (see Example 6).

More particularly, in one embodiment of the present invention, as a result of performing metagenomic analysis on bacteria-derived extracellular vesicles at an order level, the content of extracellular vesicles derived from bacteria belonging to the order YS2, the order *Turicibacterales,* the order *Bifidobacteriales,* the order *Bacteroidales,* the order *Enterobacteriales,* the order *Rhodobacterales,* the order *Gemellales,* the order *Flavobacteriales,* the order *Caulobacterales,* the order *Neisseriales,* the order *Sphingobacteriales,* the order *Deinococcales,* the order *Pseudomonadales,* the order *Rhodocyclales,* the order *Xanthomonadales,* the order *Fusobacteriales,* the order *Actinomycetales,* the order *Sphingomonadales,* the order *Verrucomicrobiales,* the order *Saprospirales,* the order *Rhizobiales,* the order *Bacillales,* the order *Streptophyta,* the order *Cytophagales,* the order *Thermales,* the order *Fimbriimonadales,* the order CW040, the order *Rickettsiales,* the order RB41, the order *Alteromonadales,* the order JG30-KF-CM45, the order I025, the order *Aeromonadales,* and the order *Solibacterales* was significantly different between lung cancer patients and asthma patients (see Example 6).

More particularly, in one embodiment of the present invention, as a result of performing metagenomic analysis on bacteria-derived extracellular vesicles at a family level, the content of extracellular vesicles derived from bacteria belonging to the family *Helicobacteraceae,* the family *Bacteroidaceae,* the family *Turicibacteraceae,* the family *Veillonellaceae,* the family *Bifidobacteriaceae,* the family *Barnesiellaceae,* the family *Rikenellaceae,* the family *Clostridiaceae,* the family *Odoribacteraceae,* the family *Enterobacteriaceae,* the family *Porphyromonadaceae,* the family *Gemellaceae,* the family *Weeksellaceae,* the family *Carnobacteriaceae,* the family *Leptotrichiaceae,* the family *Moraxellaceae,* the family *Caulobacteraceae,* the family *Erythrobacteraceae,* the family *Hyphomicrobiaceae,* the family *Neisseriaceae,* the family *Sphingobacteriaceae,* the family *Deinococcaceae,* the family *Aerococcaceae,* the family *Bartonellaceae,* the family *Micrococcaceae,* the family *Flavobacteriaceae,* the family *Burkholderiaceae,* the family *Lactobacillaceae,* the family *Dietziaceae,* the family *Rhodocyclaceae,* the family *Xanthomonadaceae,* the family *Geodermatophilaceae,* the family *Actinomycetaceae,* the family *Methylobacteriaceae,* the family *Pseudomonadaceae,* the family *Corynebacteriaceae,* the family *Staphylococcaceae,* the family *Nocardioidaceae,* the family *Verrucomicrobiaceae,* the family *Sphingomonadaceae,* the family *Mycobacteriaceae,* the family *Tissierellaceae,* the family *Chitinophagaceae,* the family *Intrasporangiaceae,* the family *Propionibacteriaceae,* the family *Aurantimonadaceae,* the family *Planococcaceae,* the family *Fusobacteriaceae,* the family *Bradyrhizobiaceae,* the family *Nocardiaceae,* the family *Dermabacteraceae,* the family *Bacillaceae,* the family *Thermaceae,* the family Ellin6075, the family *Brevibacteriaceae,* the family *Microbacteriaceae,* the family *Rhodospirillaceae,* the family *Cytophagaceae,* the family *Fimbriimonadaceae,* the family *Dermacoccaceae,* the family *Chromatiaceae,* the family *Rhizobiaceae,* the family *Gordonžaceae,* the family *mitochondria,* the family *Pseudonocardiaceae,* the family *Exiguobacteraceae,* the family *Shewanellaceae,* the family F16, the family Rs-045, and the family *Aeromonadaceae* was significantly different between lung cancer patients and asthma patients (see Example 6).

More particularly, in one embodiment of the present invention, as a result of performing metagenomic analysis on bacteria-derived extracellular vesicles at a genus level, the content of extracellular vesicles derived from bacteria belonging to the genus *Trabulsiella,* the genus *Enterobacter,* the genus *Veillonella,* the genus *Bifidobacterium,* the genus *Lachnospira,* the genus *Comamonas,* the genus *Bacteroides,* the genus *Turicibacter,* the genus *Sutterella,* the genus *Klebsiella,* the genus SMB53, the genus *Roseburia,* the genus *Dialister,* the genus *Ruminococcus,* the genus *Parabacteroides,* the genus *Butyricimonas,* the genus *Odoribacter,* the genus *Eubacterium,* the genus *Dorea,* the genus *Enhydrobacter,* the genus *Granulicatella,* the genus *Chryseobacterium,* the genus *Porphyromonas,* the genus *Coprococcus,* the genus *Peptoniphilus,* the genus *Microbispora,* the genus *Deinococcus,* the genus *Acinetobacter,* the genus *Aerococcus,* the genus *Actinomyces,* the genus *Brevundimonas,* the genus *Blastomonas,* the genus *Citrobacter,* the genus *Lactobacillus,* the genus *Stenotrophomonas,* the genus *Corynebacterium,* the genus *Pseudomonas,* the genus *Lautropia,* the genus *Akkermansia,* the genus *Staphylococcus,* the genus *Bacillus,* the genus *Sphingobacterium,* the genus *Anaerococcus,* the genus *Neisseria,* the genus *Leptotrichia,* the genus *Mycobacterium,* the genus *Kocuria,* the genus *Methylobacterium,* the genus *Propionibacterium,* the genus *Hymenobacter,* the genus *Sphingomonas,* the genus *Fusobacterium,* the genus *Brachybacterium,* the genus *Rhodococcus,* the genus *Micrococcus,* the genus *Kaistobacter,* the genus *Finegoldia,* the genus *Rubellimicrobium,* the genus *Brevibacterium,* the genus *Agrobacterium,* the genus *Dietzia,* the genus *Fimbriimonas,* the genus *Flavobacterium,* the genus *Dermacoccus,* the genus *Skermanella,* the genus *Novosphingobium,* the genus *Gordonia,* the genus *Rheinheimera,* the genus *Achromobacter,* the genus *Hydrogenophilus,* the genus *Thermus,* the genus *Exiguobacterium,* the genus *Shewanella,* the genus *Ralstonia,* and the genus *Alkanindiges* was significantly different between lung cancer patients and asthma patients (see Example 6).

From the above-described example results, it was confirmed that bacteria-derived extracellular vesicles exhibiting a significant change in content in lung cancer patients compared to normal individuals, COPD patients, and asthma patients, are identified by performing metagenomic analysis on bacteria-derived extracellular vesicles isolated from blood, and lung cancer may be diagnosed by analyzing an increase or decrease in the content of bacteria-derived extracellular vesicles at each level through metagenomic analysis.

Hereinafter, the present invention will be described with reference to exemplary examples to aid in understanding of the present invention. However, these examples are provided only for illustrative purposes and are not intended to limit the scope of the present invention.

### [Examples]

### Example 1. Analysis of In Vivo Absorption, Distribution, and Excretion Patterns of Intestinal Bacteria and Bacteria-Derived Extracellular Vesicles

To evaluate whether intestinal bacteria and bacteria-derived extracellular vesicles are systematically absorbed through the gastrointestinal tract, an experiment was conducted using the following method. More particularly, 50 µg of each of intestinal bacteria and the bacteria-derived extracellular vesicles (EVs), labeled with fluorescence, were orally administered to the gastrointestinal tracts of mice, and fluorescence was measured at 0 h, and after 5 min, 3 h, 6 h, and 12 h. As a result of observing the entire images of mice, as illustrated in FIG. 1A, the bacteria were not systematically absorbed when administered, while the bacteria-derived EVs were systematically absorbed at 5 min after administration, and, at 3 h after administration, fluorescence was strongly observed in the bladder, from which it was confirmed that the EVs were excreted via the urinary system, and were present in the bodies up to 12 h after administration.

After intestinal bacteria and intestinal bacteria-derived extracellular vesicles were systematically absorbed, to evaluate a pattern of invasion of intestinal bacteria and the bacteria-derived EVs into various organs in the human body after being systematically absorbed, 50 µg of each of the bacteria and bacteria-derived EVs, labeled with fluorescence, were administered using the same method as that used above, and then, at 12 h after administration, blood, the heart, the lungs, the liver, the kidneys, the spleen, adipose tissue, and muscle were extracted from each mouse. As a result of observing fluorescence in the extracted tissues, as illustrated in FIG. 1B, it was confirmed that the intestinal bacteria were not absorbed into each organ, while the bacteria-derived EVs were distributed in the blood, heart, lungs, liver, kidneys, spleen, adipose tissue, and muscle.

### Example 2. Vesicle Isolation and DNA Extraction from Blood

To isolate vesicles and extract DNA, from blood, first, blood was added to a 10 ml tube and centrifuged at 3,500 x g and 4 □ for 10 min to precipitate a suspension, and only a supernatant was collected, which was then placed in a new 10 ml tube. The collected supernatant was filtered using a 0.22 µm filter to remove bacteria and impurities, and then placed in centripreigugal filters (50 kD) and centrifuged at 1500 x g and 4□ for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 □ for 3 hours by using a Type 90ti rotor to remove a supernatant, and the agglomerated pellet was dissolved with phosphate-buffered saline (PBS), thereby obtaining vesicles.

100 µl of the vesicles isolated from the blood according to the above-described method was boiled at 100 □ to allow the internal DNA to come out of the lipid and then cooled on ice for 5 minutes. Next, the resulting vesicles were centrifuged at 10,000 x g and 4 □ for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below.

**[Table 1]**

| primer | | sequence | SEQ ID NO. |
|---|---|---|---|
| 16S rDNA | 16S_V3_F | 5'-TCGTCGGCAGCGTCAGATGTGTATAAG AGACAGCCTACGGGNGGCWGCAG-3' | 1 |
| | 16S_V4_R | 5'-GTCTCGTGGGCTCGGAGATGTGTATAA GAGACAGGACTACHVGGGTATCTAATCC -3 | 2 |

### Example 3. Metagenomic Analysis Using DNA Extracted from Blood

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (29 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 4. Lung Cancer Diagnostic Model Based on Metagenomic Analysis of Bacteria-Derived Extracellular Vesicles Isolated from Normal Individual-Derived Blood and Lung Cancer Patient-Derived Blood

Extracellular vesicles were isolated from blood samples of 318 lung cancer patients and 234 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

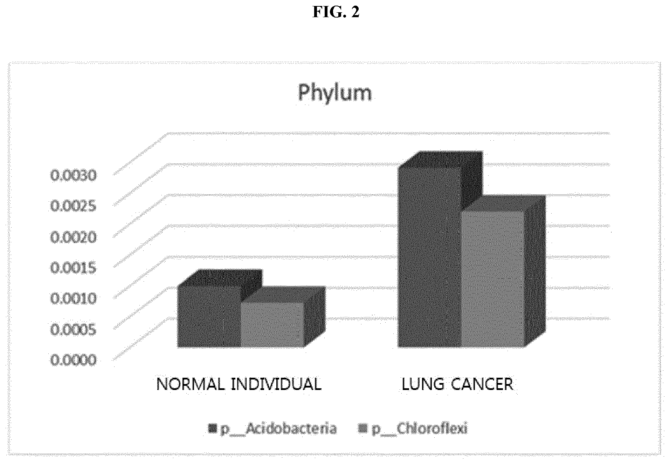

As a result of analyzing bacteria-derived extracellular vesicles in blood at a phylum level, a diagnostic model developed using, as a biomarker, one or more bacteria selected from the phylum *Acidobacteria* and the phylum *Chloroflexi* exhibited significant diagnostic performance for lung cancer (see Table 2 and FIG. 2).

**[Table 2]**

| | Normal Individual | | Lung Cancer | | t-test | | Training Set | | | Testing Set | | |
|---|---|---|---|---|---|---|---|---|---|---|---|---|
| Taxon | Mean | SD | Mean | SD | p-val ue | fold | AUC | sensit ivity | speci ficity | AUC | sensit ivity | sp ec ifi ci ty |
| p_Acid obacteri a | 0.001 0 | 0.003 4 | 0.002 9 | 0.008 4 | 0.000 2 | 2.92 | 0.73 | 0.79 | 0.53 | 0.71 | 0.76 | 0. 5 5 |
| p_Chlo roflexi | 0.000 7 | 0.003 0 | 0.002 2 | 0.004 7 | 0.000 0 | 3.03 | 0.72 | 0.78 | 0.54 | 0.76 | 0.74 | 0. 6 1 |

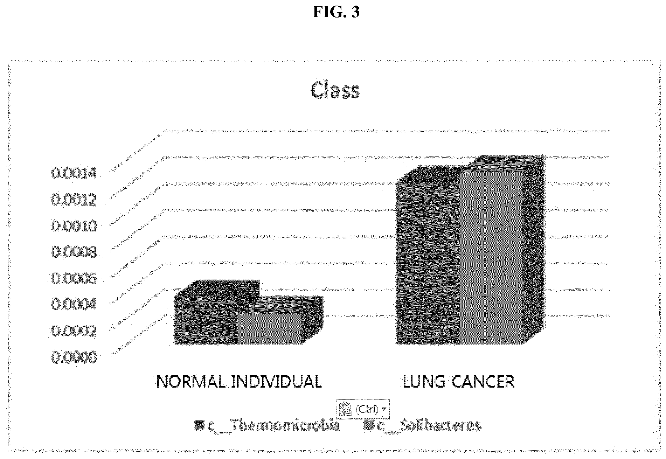

As a result of analyzing bacteria-derived extracellular vesicles in blood at a class level, a diagnostic model developed using, as a biomarker, one or more bacteria selected from the class *Thermomicrobia* and the class *Solibacteres* exhibited significant diagnostic performance for lung cancer (see Table 3 and FIG. 3).

**[Table 3]**

| | Normal Individual | | Lung Cancer | | t-test | | Training Set | | | Testing Set | | |
|---|---|---|---|---|---|---|---|---|---|---|---|---|
| Taxon | Mea n | SD | Mea n | SD | p-val uc | fold | AU C | sensiti vity | specifi city | AUC | sensiti vity | sp eci fic ity |
| c_Ther momicr obia | 0.00 04 | 0.00 14 | 0.00 12 | 0.00 33 | 0.00 00 | 3.38 | 0.72 | 0.77 | 0.54 | 0.74 | 0.75 | 0. 64 |
| c_Soli bactcrcs | 0.00 02 | 0.00 18 | 0.00 13 | 0.00 51 | 0.00 05 | 5.43 | 0.73 | 0.81 | 0.55 | 0.71 | 0.76 | 0. 53 |

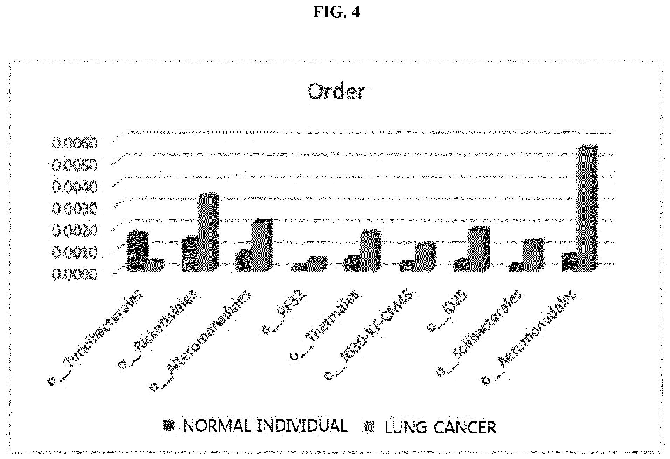

As a result of analyzing bacteria-derived extracellular vesicles in blood at an order level, a diagnostic model developed using, as a biomarker, one or more bacteria selected from the order *Turicibacterales,* the order *Rickettsiales,* the order *Alteromonadales,* the order RF32, the order *Thermales,* the order JG30-KF-CM45, the order I025, the order *Solibacterales,* and the order *Aeromonadales* exhibited significant diagnostic performance for lung cancer (see Table 4 and FIG. 4).

**[Table 4]**

| | Normal Individual | | Lung Cancer | | t-test | | Training Set | | | Testing Set | | |
|---|---|---|---|---|---|---|---|---|---|---|---|---|
| Taxon | Mea n | SD | Mea n | SD | p-val ue | fold | AU C | sensiti vity | specifi city | AU C | sensiti vity | sp ec ifi cit y |
| ο_Turici bactcralcs | 0.001 7 | 0.003 3 | 0.000 4 | 0.001 7 | 0.000 0 | 0.25 | 0.7 6 | 0.75 | 0.63 | 0.74 | 0.69 | 0. 70 |
| o_Ricke ttsiales | 0.001 4 | 0.005 3 | 0.003 4 | 0.008 9 | 0.000 6 | 2.36 | 0.7 0 | 0.75 | 0.54 | 0.72 | 0.71 | 0. 58 |
| ο_Alter omonadal es | 0.000 8 | 0.002 3 | 0.002 2 | 0.008 1 | 0.003 2 | 2.71 | 0.7 0 | 0.76 | 0.56 | 0.72 | 0.69 | 0. 59 |
| o_RF32 | 0.000 2 | 0.000 8 | 0.000 5 | 0.002 1 | 0.008 2 | 3.07 | 0.7 0 | 0.77 | 0.56 | 0.72 | 0.70 | 0. 57 |
| o_Ther males | 0.000 6 | 0.002 2 | 0.001 7 | 0.003 9 | 0.000 0 | 3.10 | 0.7 1 | 0.78 | 0.56 | 0.75 | 0.76 | 0. 59 |
| o_JG30-KF-CM4 5 | 0.000 3 | 0.001 4 | 0.001 1 | 0.003 3 | 0.000 0 | 3.49 | 0.7 2 | 0.78 | 0.55 | 0.74 | 0.75 | 0. 64 |
| o_1025 | 0.000 4 | 0.002 1 | 0.001 9 | 0.005 9 | 0.000 0 | 4.36 | 0.7 3 | 0.81 | 0.56 | 0.72 | 0.73 | 0. 55 |
| ο_Solib acterales | 0.000 2 | 0.001 8 | 0.001 3 | 0.005 1 | 0.000 5 | 5.43 | 0.7 3 | 0.81 | 0.55 | 0.71 | 0.76 | 0. 53 |
| o_Aero monadale s | 0.000 7 | 0.002 9 | 0.005 6 | 0.032 0 | 0.007 7 | 7.88 | 0.7 1 | 0.79 | 0.55 | 0.72 | 0.72 | 0. 55 |

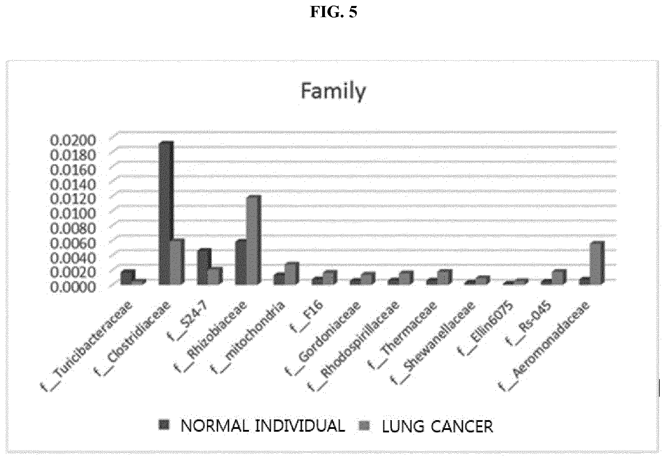

As a result of analyzing bacteria-derived extracellular vesicles in blood at a family level, a diagnostic model developed using, as a biomarker, one or more bacteria selected from the family *Turicibacteraceae,* the family *Clostridiaceae,* the family S24-7, the family *Rhizobiaceae,* the family *mitochondria,* the family F16, the family *Gordonžaceae,* the family *Rhodospirillaceae,* the family *Thermaceae,* the family *Shewanellaceae,* the family Ellin6075, the family Rs-045, and the family *Aeromonadaceae* exhibited significant diagnostic performance for lung cancer (see Table 5 and FIG. 5).

**[Table 5]**

| | Normal Individual | | Lung Cancer | | t-test | | Training Set | | | Testing Set | | |
|---|---|---|---|---|---|---|---|---|---|---|---|---|
| Taxon | Mea n | SD | Mea n | SD | p-val ue | fol d | AU C | sensiti vity | specifi city | AU C | sensiti vity | sp ec ifi cit y |
| f Turicib acteraceae | 0.001 7 | 0.003 3 | 0.000 4 | 0.001 7 | 0.000 0 | 0.2 5 | 0.7 6 | 0.75 | 0.63 | 0.74 | 0.69 | 0. 70 |
| f_Clostri diaceae | 0.019 1 | 0.045 0 | 0.005 9 | 0.007 8 | 0.000 0 | 0.3 1 | 0.7 3 | 0.75 | 0.59 | 0.76 | 0.71 | 0. 64 |
| f_S24-7 | 0.004 6 | 0.011 9 | 0.002 0 | 0.004 0 | 0.000 0 | 0.4 4 | 0.6 9 | 0.75 | 0.55 | 0.71 | 0.69 | 0. 59 |
| f_Rhizob iaceae | 0.005 8 | 0.008 2 | 0.011 8 | 0.015 0 | 0.000 0 | 2.0 2 | 0.7 2 | 0.77 | 0.52 | 0.77 | 0.75 | 0. 63 |
| f_mitoch ondria | 0.001 3 | 0.005 3 | 0.002 7 | 0.008 1 | 0.006 5 | 2.1 2 | 0.6 9 | 0.75 | 0.56 | 0.72 | 0.69 | 0. 59 |
| f_F16 | 0.000 7 | 0.003 2 | 0.001 6 | 0.004 0 | 0.001 2 | 2.2 3 | 0.7 1 | 0.78 | 0.56 | 0.72 | 0.72 | 0. 55 |
| f_Gordon iaceae | 0.000 5 | 0.002 4 | 0.001 3 | 0.004 8 | 0.004 8 | 2.6 3 | 0.7 0 | 0.78 | 0.56 | 0.73 | 0.71 | 0. 59 |
| f_Rhodos pirillaceae | 0.000 6 | 0.002 2 | 0.001 5 | 0.006 2 | 0.008 8 | 2.7 0 | 0.7 0 | 0.77 | 0.58 | 0.71 | 0.69 | 0. 58 |
| f_Therma ceae | 0.000 6 | 0.002 2 | 0.001 7 | 0.003 9 | 0.000 0 | 3.1 0 | 0.7 1 | 0.78 | 0.56 | 0.75 | 0.76 | 0. 59 |
| f_Shewa nellaceae | 0.000 3 | 0.001 2 | 0.000 9 | 0.004 0 | 0.009 3 | 3.3 0 | 0.7 0 | 0.76 | 0.56 | 0.72 | 0.70 | 0. 58 |
| f_Ellin60 75 | 0.000 1 | 0.000 9 | 0.000 5 | 0.001 8 | 0.002 0 | 3.7 3 | 0.7 1 | 0.77 | 0.56 | 0.73 | 0.71 | 0. 57 |
| f_Rs-045 | 0.000 4 | 0.002 1 | 0.001 7 | 0.005 8 | 0.000 1 | 4.2 2 | 0.7 2 | 0.81 | 0.54 | 0.72 | 0.73 | 0. 55 |
| f_Aerom onadaceae | 0.000 7 | 0.002 9 | 0.005 6 | 0.032 0 | 0.007 5 | 8.1 5 | 0.7 1 | 0.79 | 0.55 | 0.72 | 0.72 | 0. 55 |

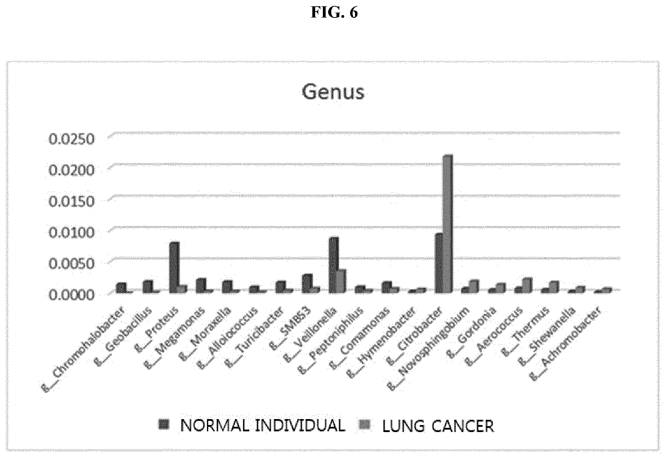

As a result of analyzing bacteria-derived extracellular vesicles in blood at a genus level, a diagnostic model developed using, as a biomarker, one or more bacteria selected from the genus *Chromohalobacter,* the genus *Geobacillus,* the genus *Proteus,* the genus *Megamonas,* the genus *Moraxella,* the genus *Alloiococcus,* the genus *Turicibacter,* the genus SMB53, the genus *Veillonella,* the genus *Peptoniphilus,* the genus *Comamonas,* the genus *Hymenobacter,* the genus *Citrobacter,* the genus *Novosphingobium,* the genus *Gordonia,* the genus *Aerococcus,* the genus *Thermus,* the genus *Shewanella,* and the genus *Achromobacter* exhibited significant diagnostic performance for lung cancer (see Table 6 and FIG. 6).

**[Table 6]**

| | Normal Individual | | Lung Cancer | | t-test | | Training Set | | | Testing Set | | |
|---|---|---|---|---|---|---|---|---|---|---|---|---|
| Taxon | Mean | SD | Mean | SD | p-val ue | fold | A U C | sensiti vity | specif icity | AU C | sensiti vity | sp ec ifi cit y |
| g_Chrom ohalobacte r | 0.001 4 | 0.007 5 | 0.000 0 | 0.000 0 | 0.000 1 | 0.00 | 0.7 2 | 0.72 | 0.63 | 0.75 | 0.69 | 0. 69 |
| g_Geoba cillus | 0.001 8 | 0.006 2 | 0.000 1 | 0.000 6 | 0.000 0 | 0.06 | 0.7 0 | 0.74 | 0.59 | 0.71 | 0.66 | 0. 66 |
| g_Proteus | 0.007 9 | 0.023 1 | 0.001 0 | 0.002 5 | 0.000 0 | 0.13 | 0.7 6 | 0.76 | 0.62 | 0.75 | 0.69 | 0. 69 |
| g_Mega monas | 0.002 1 | 0.009 7 | 0.000 3 | 0.001 4 | 0.000 1 | 0.14 | 0.7 1 | 0.76 | 0.60 | 0.71 | 0.65 | 0. 66 |
| g_Morax ella | 0.001 8 | 0.010 9 | 0.000 3 | 0.001 8 | 0.005 5 | 0.15 | 0.7 0 | 0.76 | 0.57 | 0.71 | 0.65 | 0. 63 |
| g_Alloioc occus | 0.000 9 | 0.004 1 | 0.000 2 | 0.000 8 | 0.000 1 | 0.17 | 0.7 1 | 0.75 | 0.59 | 0.71 | 0.67 | 0. 66 |
| g_Turicib acter | 0.001 7 | 0.003 3 | 0.000 4 | 0.001 7 | 0.000 0 | 0.25 | 0.7 6 | 0.75 | 0.63 | 0.74 | 0.69 | 0. 70 |
| g_SMB5 3 | 0.002 8 | 0.007 8 | 0.000 7 | 0.001 5 | 0.000 0 | 0.26 | 0.7 2 | 0.75 | 0.59 | 0.74 | 0.68 | 0. 70 |
| g_Veillon ella | 0.008 6 | 0.015 5 | 0.003 5 | 0.005 4 | 0.000 0 | 0.41 | 0.7 1 | 0.74 | 0.59 | 0.74 | 0.70 | 0. 65 |
| g_Pepton iphilus | 0.000 9 | 0.003 6 | 0.000 4 | 0.001 3 | 0.003 4 | 0.41 | 0.7 0 | 0.76 | 0.56 | 0.71 | 0.66 | 0. 63 |
| g_Coma monas | 0.001 6 | 0.003 8 | 0.000 7 | 0.002 3 | 0.000 1 | 0.45 | 0.7 1 | 0.75 | 0.58 | 0.71 | 0.68 | 0. 67 |
| g_Hymen obacter | 0.000 3 | 0.001 0 | 0.000 6 | 0.002 1 | 0.009 5 | 2.26 | 0.7 0 | 0.76 | 0.56 | 0.71 | 0.69 | 0. 57 |
| g_Citroba cter | 0.009 3 | 0.014 6 | 0.021 8 | 0.022 0 | 0.000 0 | 2.33 | 0.7 4 | 0.81 | 0.54 | 0.81 | 0.85 | 0. 61 |
| g_Novos phingobiu m | 0.000 7 | 0.002 7 | 0.001 9 | 0.004 3 | 0.000 1 | 2.58 | 0.7 3 | 0.81 | 0.53 | 0.73 | 0.75 | 0. 66 |
| g_Gordo nia | 0.000 5 | 0.002 4 | 0.001 3 | 0.004 8 | 0.004 7 | 2.64 | 0.7 0 | 0.78 | 0.56 | 0.73 | 0.71 | 0. 59 |
| g_Acroco ccus | 0.000 8 | 0.002 5 | 0.002 2 | 0.007 5 | 0.001 3 | 2.86 | 0.7 1 | 0.79 | 0.54 | 0.70 | 0.72 | 0. 55 |
| g_Therm us | 0.000 6 | 0.002 2 | 0.001 7 | 0.003 8 | 0.000 0 | 3.02 | 0.7 1 | 0.78 | 0.56 | 0.74 | 0.76 | 0. 58 |
| g_Shewa nella | 0.000 3 | 0.001 2 | 0.000 9 | 0.004 0 | 0.009 3 | 3.30 | 0.7 0 | 0.76 | 0.56 | 0.72 | 0.70 | 0. 58 |
| g_Achro mobacter | 0.000 2 | 0.001 1 | 0.000 6 | 0.002 8 | 0.006 0 | 3.56 | 0.7 0 | 0.76 | 0.56 | 0.72 | 0.70 | 0. 56 |

### Example 5. Lung Cancer Diagnostic Model Based on Metagenomic Analysis of Bacteria-Derived Extracellular Vesicles Isolated from COPD Patient-Derived Blood and Lung Cancer Patient-Derived Blood

Extracellular vesicles were isolated from blood samples of 319 lung cancer patients and 208 COPD patients, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an AUC, sensitivity, and specificity, which are diagnostic performance indexes, were calculated by logistic regression analysis.

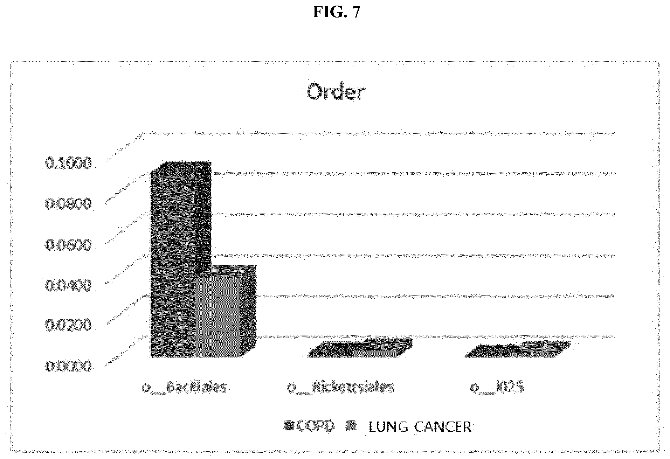

As a result of analyzing bacteria-derived extracellular vesicles in blood at an order level, a diagnostic model developed using, as a biomarker, one or more bacteria selected from the order *Bacillales,* the order *Rickettsiales,* and the order I025 exhibited significant diagnostic performance for lung cancer (see Table 7 and FIG. 7).

**[Table 7]**

| | COPD | | Lung Cancer | | t-test | | Training Set | | | Testing Set | | |
|---|---|---|---|---|---|---|---|---|---|---|---|---|
| Taxon | Mean | SD | Mean | SD | p-val ue | fold | AUC | sensiti vity | specifi city | AUC | sensiti vity | s p e ci fi ci ty |
| ο_Ba cillales | 0.090 3 | 0.074 0 | 0.039 4 | 0.050 9 | 0.000 0 | 0.4 4 | 0.82 | 0.50 | 0.89 | 0.83 | 0.46 | 0. 8 5 |
| o_Ri ckettsi ales | 0.001 5 | 0.002 7 | 0.003 4 | 0.008 9 | 0.000 7 | 2.1 8 | 0.58 | 0.00 | 0.99 | 0.60 | 0.00 | 1. 0 0 |
| o_I02 5 | 0.000 4 | 0.001 2 | 0.001 9 | 0.005 9 | 0.000 0 | 5.3 3 | 0.60 | 0.05 | 0.95 | 0.65 | 0.05 | 0. 9 7 |

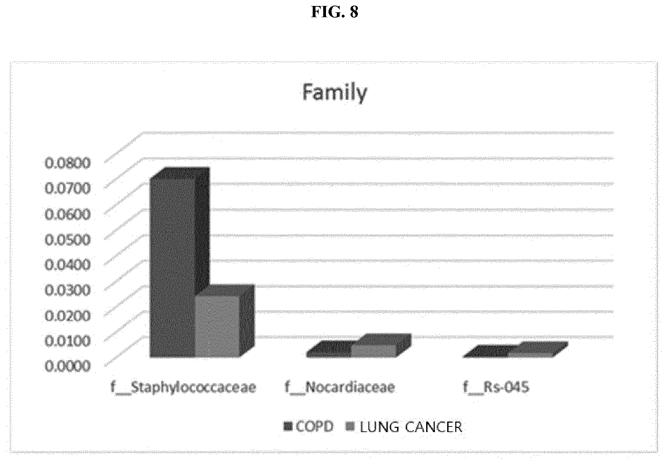

As a result of analyzing bacteria-derived extracellular vesicles in blood at a family level, a diagnostic model developed using, as a biomarker, one or more bacteria selected from the family *Staphylococcaceae,* the family *Nocardiaceae,* and the family Rs-045 exhibited significant diagnostic performance for lung cancer (see Table 8 and FIG. 8).

**[Table 8]**

| | COPD | | Lung Cancer | | t-test | | Training Set | | | Testing Set | | |
|---|---|---|---|---|---|---|---|---|---|---|---|---|
| Taxon | Mea n | SD | Mea n | SD | p-val ue | fold | AUC | sensit ivity | specif icity | AUC | sensiti vity | sp ec ifi ci ty |
| f_Staphyl ococcacea e | 0.07 00 | 0.07 11 | 0.02 42 | 0.04 37 | 0.00 00 | 0.35 | 0.85 | 0.53 | 0.94 | 0.84 | 0.52 | 0. 8 6 |
| f_Nocard iaceae | 0.00 20 | 0.00 28 | 0.00 48 | 0.01 03 | 0.00 00 | 2.35 | 0.61 | 0.19 | 0.80 | 0.61 | 0.18 | 0. 8 3 |
| f_Rs-045 | 0.00 03 | 0.00 11 | 0.00 17 | 0.00 58 | 0.00 00 | 5.49 | 0.60 | 0.06 | 0.95 | 0.64 | 0.07 | 0. 9 5 |

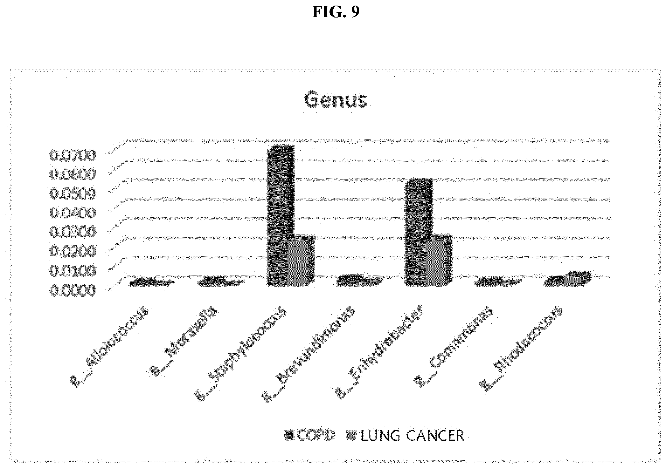

As a result of analyzing bacteria-derived extracellular vesicles in blood at a genus level, a diagnostic model developed using, as a biomarker, one or more bacteria selected from the genus *Alloiococcus,* the genus *Moraxella,* the genus *Staphylococcus,* the genus *Brevundimonas,* the genus *Enhydrobacter,* the genus *Comamonas,* and the genus *Rhodococcus* exhibited significant diagnostic performance for lung cancer (see Table 9 and FIG. 9).

**[Table 9]**

| | COPD | | Lung Cancer | | t-test | | Training Set | | | Testing Set | | |
|---|---|---|---|---|---|---|---|---|---|---|---|---|
| Taxon | Mean | SD | Mean | SD | p-val ue | fold | AUC | sensit ivity | speci ficity | AUC | sensit ivity | sp ec ifi ci ty |
| g_Alloio coccus | 0.001 1 | 0.003 7 | 0.000 2 | 0.000 8 | 0.000 3 | 0.14 | 0.61 | 0.13 | 0.97 | 0.64 | 0.16 | 0. 9 6 |
| g_Morax ella | 0.001 9 | 0.005 4 | 0.000 3 | 0.001 8 | 0.000 1 | 0.15 | 0.64 | 0.14 | 0.98 | 0.63 | 0.13 | 0. 9 8 |
| g_Staph ylococcus | 0.069 4 | 0.071 1 | 0.023 3 | 0.043 5 | 0.000 0 | 0.34 | 0.85 | 0.53 | 0.92 | 0.84 | 0.52 | 0. 8 6 |
| g_Brevu ndimonas | 0.003 1 | 0.005 0 | 0.001 2 | 0.003 4 | 0.000 0 | 0.40 | 0.66 | 0.19 | 0.93 | 0.71 | 0.20 | 0. 9 5 |
| g_Enhyd robacter | 0.052 5 | 0.047 8 | 0.023 6 | 0.029 3 | 0.000 0 | 0.45 | 0.77 | 0.35 | 0.92 | 0.79 | 0.39 | 0. 8 8 |
| g_Coma monas | 0.001 5 | 0.002 6 | 0.000 7 | 0.002 3 | 0.000 3 | 0.47 | 0.70 | 0.18 | 0.95 | 0.63 | 0.18 | 0. 9 1 |
| g_Rhodo coccus | 0.002 0 | 0.002 8 | 0.004 7 | 0.010 2 | 0.000 0 | 2.31 | 0.61 | 0.19 | 0.80 | 0.61 | 0.18 | 0. 8 3 |

### Example 6. Lung Cancer Diagnostic Model Based on Metagenomic Analysis of Bacteria-Derived Extracellular Vesicles Isolated from Asthma Patient-Derived Blood and Lung Cancer Patient-Derived Blood

Extracellular vesicles were isolated from blood samples of 308 lung cancer patients and 277 asthma patients, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an AUC, sensitivity, and specificity, which are diagnostic performance indexes, were calculated by logistic regression analysis.

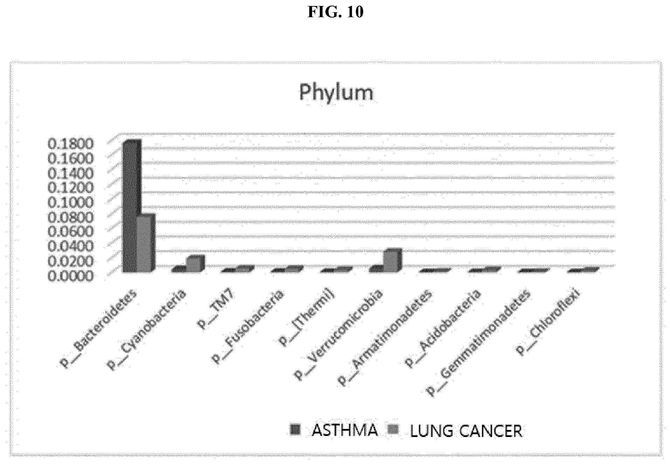

As a result of analyzing bacteria-derived extracellular vesicles in blood at a phylum level, a diagnostic model developed using, as a biomarker, one or more bacteria selected from the phylum *Bacteroidetes,* the phylum *Cyanobacteria,* the phylum TM7, the phylum *Fusobacteria,* the phylum *Thermi,* the phylum *Verrucomicrobia,* the phylum *Armatimonadetes,* the phylum *Acidobacteria,* the phylum *Gemmatimonadetes,* and the phylum *Chloroflexi* exhibited significant diagnostic performance for lung cancer (see Table 10 and FIG. 10).

**[Table 10]**

| | Asthma | | Lung Cancer | | t-test | | Training Set | | | Test Set | | |
|---|---|---|---|---|---|---|---|---|---|---|---|---|
| Taxon | Mea n | SD | Mea n | SD | p-val ue | fold | Auc | sensi tivity | speci ficity | Auc | sensi tivity | s p c ci fi ci ty |
| p_Bacteroi detes | 0.17 62 | 0.04 99 | 0.07 57 | 0.03 70 | 0.00 00 | 0.43 | 0.97 | 0.88 | 0.93 | 0.96 | 0.90 | 0. 9 1 |
| p_Cyanoba cteria | 0.00 43 | 0.01 19 | 0.01 89 | 0.03 19 | 0.00 00 | 4.39 | 0.89 | 0.70 | 0.86 | 0.85 | 0.75 | 0. 8 0 |
| p_TM7 | 0.00 10 | 0.00 29 | 0.00 51 | 0.00 80 | 0.00 00 | 4.98 | 0.87 | 0.65 | 0.90 | 0.78 | 0.69 | 0. 7 4 |
| p_Fusobac tcria | 0.00 09 | 0.00 29 | 0.00 47 | 0.00 95 | 0.00 00 | 5.04 | 0.85 | 0.63 | 0.89 | 0.84 | 0.68 | 0. 8 4 |
| p_[Thermi ] | 0.00 06 | 0.00 19 | 0.00 31 | 0.00 65 | 0.00 00 | 5.25 | 0.83 | 0.62 | 0.88 | 0.80 | 0.65 | 0. 8 2 |
| p_Verruco microbia | 0.00 53 | 0.00 60 | 0.02 77 | 0.02 36 | 0.00 00 | 5.25 | 0.91 | 0.79 | 0.82 | 0.87 | 0.78 | 0. 7 7 |
| p_Armati monadetes | 0.00 01 | 0.00 04 | 0.00 09 | 0.00 51 | 0.00 46 | 8.82 | 0.81 | 0.62 | 0.87 | 0.76 | 0.65 | 0. 7 8 |
| p_Acidoba cteria | 0.00 03 | 0.00 11 | 0.00 29 | 0.00 84 | 0.00 00 | 8.85 | 0.83 | 0.62 | 0.89 | 0.82 | 0.63 | 0. 8 0 |
| p_Gemmat imonadetes | 0.00 01 | 0.00 02 | 0.00 05 | 0.00 19 | 0.00 00 | 10.0 4 | 0.80 | 0.62 | 0.86 | 0.77 | 0.66 | 0. 77 |
| p_Chlorofl exi | 0.00 01 | 0.00 03 | 0.00 22 | 0.00 47 | 0.00 00 | 22.4 1 | 0.84 | 0.62 | 0.91 | 0.87 | 0.63 | 0. 8 9 |

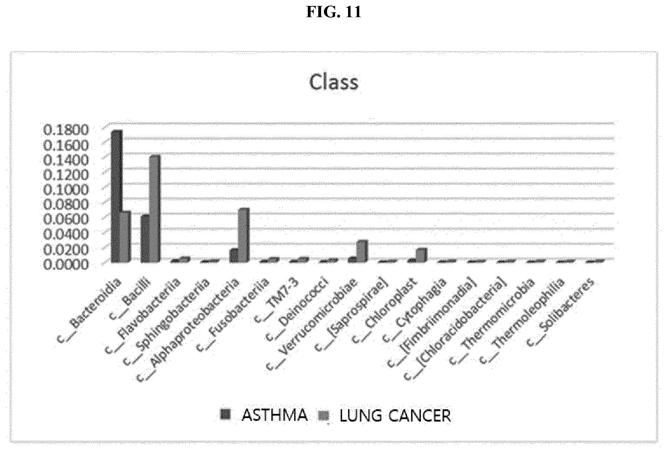

As a result of analyzing bacteria-derived extracellular vesicles in blood at a class level, a diagnostic model developed using, as a biomarker, one or more bacteria selected from the class *Bacteroidia,* the class *Bacilli,* the class *Flavobacteriia,* the class *Sphingobacteriia,* the class *Alphaproteobacteria,* the class *Fusobacteriia,* the class TM7-3, the class *Deinococci,* the class *Verrucomicrobiae,* the class *Saprospirae,* the class *Chloroplast,* the class *Cytophagia,* the class *Fimbriimonadia,* the class *Chloracidobacteria,* the class *Thermomicrobia,* the class *Thermoleophilia,* and the class *Solibacteres* exhibited significant diagnostic performance for lung cancer (see Table 11 and FIG. 11).

**[Table 11]**

| | Asthma | | Lung Cancer | | t-test | | Training Set | | | Test Set | | |
|---|---|---|---|---|---|---|---|---|---|---|---|---|
| Taxon | Mea n | SD | Mea n | SD | p-val ue | fold | AUC | sensi tivity | speci ficity | AUC | sensi tivity | s p e c if i c it y |
| c_Bacteroid ia | 0.17 36 | 0.05 14 | 0.06 61 | 0.03 80 | 0.00 00 | 0.38 | 0.97 | 0.89 | 0.94 | 0.95 | 0.88 | 0 .9 4 |
| c_Bacilli | 0.06 11 | 0.04 08 | 0.14 04 | 0.07 28 | 0.00 00 | 2.30 | 0.92 | 0.81 | 0.91 | 0.91 | 0.85 | 0 .9 1 |
| c_Flavobac teriia | 0.00 18 | 0.00 66 | 0.00 55 | 0.00 88 | 0.00 00 | 3.07 | 0.86 | 0.62 | 0.86 | 0.80 | 0.72 | 0 .8 6 |
| c_Sphingob acteriia | 0.00 04 | 0.00 11 | 0.00 14 | 0.00 42 | 0.00 00 | 3.74 | 0.81 | 0.62 | 0.85 | 0.76 | 0.66 | 0 .8 5 |
| c_Alphapro teobacteria | 0.01 63 | 0.03 23 | 0.07 00 | 0.04 53 | 0.00 00 | 4.29 | 0.95 | 0.82 | 0.91 | 0.92 | 0.85 | 0 .9 1 |
| c_Fusobact eriia | 0.00 09 | 0.00 29 | 0.00 47 | 0.00 95 | 0.00 00 | 5.04 | 0.85 | 0.63 | 0.89 | 0.84 | 0.68 | 0 .8 9 |
| c_TM7-3 | 0.00 09 | 0.00 27 | 0.00 48 | 0.00 79 | 0.00 00 | 5.19 | 0.87 | 0.66 | 0.90 | 0.77 | 0.68 | 0 .9 0 |
| c_Deinococ ci | 0.00 06 | 0.00 19 | 0.00 31 | 0.00 65 | 0.00 00 | 5.25 | 0.83 | 0.62 | 0.88 | 0.80 | 0.65 | 0 .8 8 |
| c_Verruco microbiae | 0.00 52 | 0.00 60 | 0.02 74 | 0.02 35 | 0.00 00 | 5.29 | 0.91 | 0.79 | 0.83 | 0.87 | 0.76 | 0 .8 3 |
| c_[Saprospi rac] | 0.00 02 | 0.00 06 | 0.00 10 | 0.00 34 | 0.00 00 | 5.34 | 0.81 | 0.63 | 0.88 | 0.77 | 0.63 | 0 .8 8 |
| c_Chloropl ast | 0.00 27 | 0.00 49 | 0.01 69 | 0.03 15 | 0.00 00 | 6.22 | 0.89 | 0.68 | 0.87 | 0.86 | 0.75 | 0 .8 7 |
| c_Cytopha gia | 0.00 02 | 0.00 08 | 0.00 14 | 0.00 40 | 0.00 00 | 6.73 | 0.82 | 0.62 | 0.86 | 0.77 | 0.68 | 0 .8 6 |
| | | | | | | | | | | | | |
| c_[Fimbrii monadia] | 0.00 01 | 0.00 04 | 0.00 09 | 0.00 51 | 0.00 46 | 8.81 | 0.81 | 0.62 | 0.87 | 0.76 | 0.65 | 0 .8 7 |
| c_[Chloraci dobacteria] | 0.00 01 | 0.00 05 | 0.00 10 | 0.00 52 | 0.00 15 | 11.8 8 | 0.81 | 0.62 | 0.90 | 0.76 | 0.65 | 0 .9 0 |
| c_Thermo microbia | 0.00 00 | 0.00 02 | 0.00 12 | 0.00 33 | 0.00 00 | 26.0 4 | 0.82 | 0.62 | 0.89 | 0.84 | 0.63 | 0 .8 9 |
| c_Thermole ophilia | 0.00 00 | 0.00 02 | 0.00 10 | 0.00 64 | 0.00 69 | 28.5 3 | 0.80 | 0.62 | 0.87 | 0.77 | 0.66 | 0 .8 7 |
| c_Solibacte res | 0.00 00 | 0.00 02 | 0.00 13 | 0.00 51 | 0.00 00 | 41.6 8 | 0.81 | 0.61 | 0.87 | 0.82 | 0.66 | 0 .8 7 |

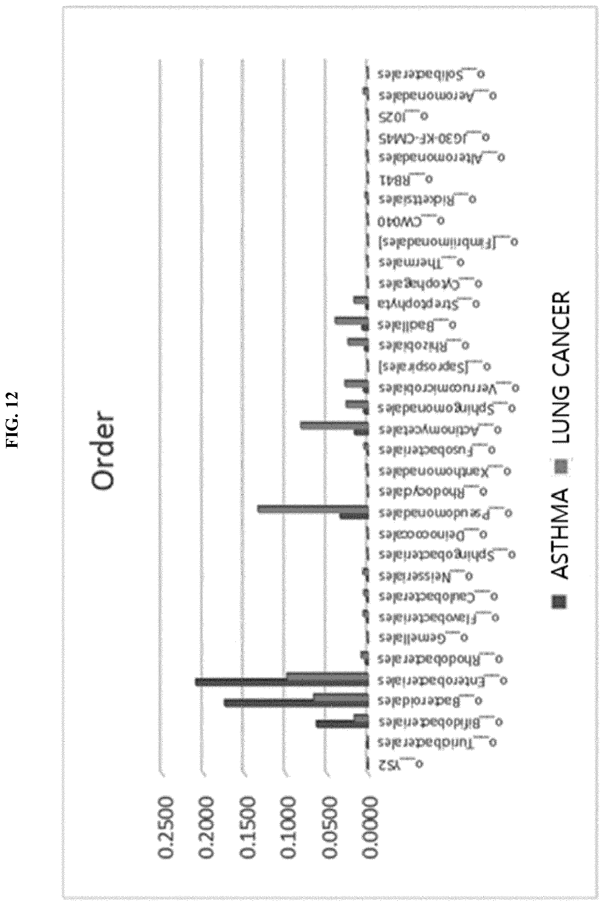

As a result of analyzing bacteria-derived extracellular vesicles in blood at an order level, a diagnostic model developed using, as a biomarker, one or more bacteria selected from the order YS2, the order *Turicibacterales,* the order *Bifidobacteriales,* the order *Bacteroidales,* the order *Enterobacteriales,* the order *Rhodobacterales,* the order *Gemellales,* the order *Flavobacteriales,* the order *Caulobacterales,* the order *Neisseriales,* the order *Sphingobacteriales,* the order *Deinococcales,* the order *Pseudomonadales,* the order *Rhodocyclales,* the order *Xanthomonadales,* the order *Fusobacteriales,* the order *Actinomycetales,* the order *Sphingomonadales,* the order *Verrucomicrobiales,* the order *Saprospirales,* the order *Rhizobiales,* the order *Bacillales,* the order *Streptophyta,* the order *Cytophagales,* the order *Thermales,* the order *Fimbriimonadales,* the order CW040, the order *Rickettsiales,* the order RB41, the order *Alteromonadales,* the order JG30-KF-CM45, the order I025, the order *Aeromonadales,* and the order *Solibacterales* exhibited significant diagnostic performance for lung cancer (see Table 12 and FIG. 12).

**[Table 12]**

| | Asthma | | Lung Cancer | | t-test | | Training Set | | | Test Set | | |
|---|---|---|---|---|---|---|---|---|---|---|---|---|
| Taxon | Mea n | SD | Mea n | SD | p-val ue | fol d | AU C | sensiti vity | specifi city | AU C | sensit ivity | spe cifi city |
| o_YS2 | 0.00 07 | 0.00 14 | 0.00 01 | 0.00 09 | 0.00 00 | 0.1 6 | 0.8 1 | 0.64 | 0.86 | 0.8 1 | 0.66 | 0.7 5 |
| o_Turicibact erales | 0.00 17 | 0.00 53 | 0.00 04 | 0.00 17 | 0.00 09 | 0.2 5 | 0.8 0 | 0.62 | 0.86 | 0.7 7 | 0.65 | 0.7 4 |
| o_Bifidobact eriales | 0.06 27 | 0.03 35 | 0.01 64 | 0.01 44 | 0.00 00 | 0.2 6 | 0.9 5 | 0.85 | 0.95 | 0.9 2 | 0.82 | 0.8 8 |
| o_Bacteroid ales | 0.17 36 | 0.05 14 | 0.06 61 | 0.03 80 | 0.00 00 | 0.3 8 | 0.9 7 | 0.89 | 0.94 | 0.9 5 | 0.88 | 0.9 2 |
| o_Enterobac teriales | 0.20 91 | 0.08 78 | 0.09 83 | 0.06 29 | 0.00 00 | 0.4 7 | 0.9 1 | 0.78 | 0.91 | 0.9 1 | 0.81 | 0.8 5 |
| o_Rhodobac terales | 0.00 34 | 0.02 37 | 0.00 80 | 0.01 25 | 0.00 92 | 2.3 6 | 0.8 0 | 0.62 | 0.86 | 0.7 7 | 0.68 | 0.7 4 |
| o_Gemellale s | 0.00 04 | 0.00 32 | 0.00 12 | 0.00 32 | 0.00 89 | 2.6 4 | 0.7 9 | 0.62 | 0.86 | 0.7 6 | 0.66 | 0.7 5 |
| o_Flavobact eriales | 0.00 18 | 0.00 66 | 0.00 55 | 0.00 88 | 0.00 00 | 3.0 7 | 0.8 6 | 0.62 | 0.86 | 0.8 0 | 0.72 | 0.8 2 |
| o_Caulobact erales | 0.00 15 | 0.00 30 | 0.00 50 | 0.00 72 | 000 00 | 3.3 1 | 0.8 5 | 0.61 | 0.89 | 0.8 0 | 0.71 | 0.7 7 |
| o_Neisserial es | 0.00 16 | 0.00 64 | 0.00 58 | 0.01 19 | 0.00 00 | 3.6 1 | 0.8 2 | 0.62 | 0.87 | 0.8 0 | 0.69 | 0.7 6 |
| ο_Sphingob acteriales | 0.00 04 | 0.00 11 | 0.00 14 | 0.00 42 | 0.00 00 | 3.7 4 | 0.8 1 | 0.62 | 0.85 | 0.7 6 | 0.66 | 0.7 4 |
| ο_Deinococ cales | 0.00 04 | 0.00 13 | 0.00 14 | 0.00 53 | 0.00 10 | 3.8 9 | 0.7 9 | 0.62 | 0.86 | 0.7 6 | 0.66 | 0.7 5 |
| ο_Pseudomo nadales | 0.03 35 | 0.04 96 | 0.13 34 | 0.07 91 | 0.00 00 | 3.9 8 | 0.9 6 | 0.87 | 0.92 | 0.9 3 | 0.87 | 0.8 6 |
| ο_Rhodocyc lales | 0.00 03 | 0.00 09 | 0.00 12 | 0.00 45 | 0.00 06 | 4.3 1 | 0.8 0 | 0.62 | 0.86 | 0.7 5 | 0.65 | 0.7 5 |
| ο_Xanthomo nadales | 0.00 06 | 0.00 12 | 0.00 25 | 0.00 59 | 0.00 00 | 4.4 7 | 0.8 1 | 0.62 | 0.88 | 0.7 7 | 0.68 | 0.7 6 |
| ο_Fusobacte riales | 0.00 09 | 0.00 29 | 0.00 47 | 0.00 95 | 0.00 00 | 5.0 4 | 0.8 5 | 0.63 | 0.89 | 0.8 4 | 0.68 | 0.8 4 |
| ο_Actinomy cetales | 0.01 60 | 0.02 18 | 0.08 13 | 0.04 58 | 0.00 00 | 5.0 7 | 0.9 7 | 0.90 | 0.92 | 0.9 6 | 0.88 | 0.8 8 |
| ο_Sphingom onadales | 0.00 49 | 0.00 82 | 0.02 61 | 0.02 54 | 0.00 00 | 5.2 7 | 0.9 1 | 0.74 | 0.88 | 0.9 1 | 0.79 | 0.8 0 |
| ο_Verrucom icrobiales | 0.00 52 | 0.00 60 | 0.02 74 | 0.02 35 | 0.00 00 | 5.2 9 | 0.9 1 | 0.79 | 0.83 | 0.8 7 | 0.76 | 0.7 7 |
| o_[Saprospir ales] | 0.00 02 | 0.00 06 | 0.00 10 | 0.00 34 | 0.00 00 | 5.3 4 | 0.8 1 | 0.63 | 0.88 | 0.7 7 | 0.63 | 0.7 3 |
| ο_Rhizobial es | 0.00 43 | 0.00 57 | 0.02 37 | 0.02 02 | 0.00 00 | 5.5 4 | 0.9 3 | 0.80 | 0.87 | 0.9 0 | 0.84 | 0.8 5 |
| o_Bacillales | 0.00 71 | 0.00 85 | 0.03 94 | 0.05 09 | 0.00 00 | 5.5 7 | 0.9 4 | 0.84 | 0.88 | 0.9 2 | 0.82 | 0.8 5 |
| o_Streptoph yta | 0.00 27 | 0.00 48 | 0.01 65 | 0.03 14 | 0.00 00 | 6.2 3 | 0.8 9 | 0.68 | 0.88 | 0.8 6 | 0.75 | 0.8 2 |
| o_Cytophag ales | 0.00 02 | 0.00 08 | 0.00 14 | 0.00 40 | 0.00 00 | 6.7 3 | 0.8 2 | 0.62 | 0.86 | 0.7 7 | 0.68 | 0.7 8 |
| ο_Thermales | 0.00 02 | 0.00 14 | 0.00 17 | 0.00 39 | 0.00 00 | 7.3 2 | 0.8 3 | 0.60 | 0.89 | 0.7 9 | 0.66 | 0.7 9 |
| o_[Fimbriim onadales] | 0.00 01 | 0.00 04 | 0.00 09 | 0.00 51 | 0.00 46 | 8.8 1 | 0.8 1 | 0.62 | 0.87 | 0.7 6 | 0.65 | 0.7 8 |
| o_CW040 | 0.00 02 | 0.00 09 | 0.00 16 | 0.00 40 | 0.00 00 | 9.4 2 | 0.8 4 | 0.62 | 0.88 | 0.7 6 | 0.66 | 0.7 5 |
| ο_Rickettsia les | 0.00 03 | 0.00 11 | 0.00 34 | 0.00 89 | 0.00 00 | 12. 76 | 0.8 6 | 0.61 | 0.90 | 0.7 6 | 0.62 | 0.7 4 |
| o_RB41 | 0.00 01 | 0.00 05 | 0.00 10 | 0.00 52 | 0.00 16 | 12. 88 | 0.8 1 | 0.62 | 0.89 | 0.7 6 | 0.65 | 0.7 6 |
| o_Alteromo nadales | 0.00 02 | 0.00 07 | 0.00 22 | 0.00 81 | 0.00 00 | 13. 64 | 0.8 2 | 0.62 | 0.86 | 0.7 9 | 0.66 | 0.7 8 |
| o_JG30-KF-CM45 | 0.0000 | 0.0002 | 0.0011 | 0.0033 | 0.0000 | 24.15 | 0.82 | 0.62 | 0.89 | 0.84 | 0.63 | 0.84 |
| o_I025 | 0.00 01 | 0.00 05 | 0.00 19 | 0.00 59 | 0.00 00 | 27. 72 | 0.8 3 | 0.62 | 0.88 | 0.7 9 | 0.66 | 0.7 8 |
| ο_Aeromona dales | 0.00 02 | 0.00 07 | 0.00 56 | 0.03 20 | 0.00 30 | 34. 45 | 0.8 2 | 0.62 | 0.87 | 0.7 8 | 0.68 | 0.7 4 |
| o_Solibacter ales | 0.00 00 | 0.00 02 | 0.00 13 | 0.00 51 | 0.00 00 | 41. 68 | 0.8 1 | 0.61 | 0.87 | 0.8 2 | 0.66 | 0.8 2 |

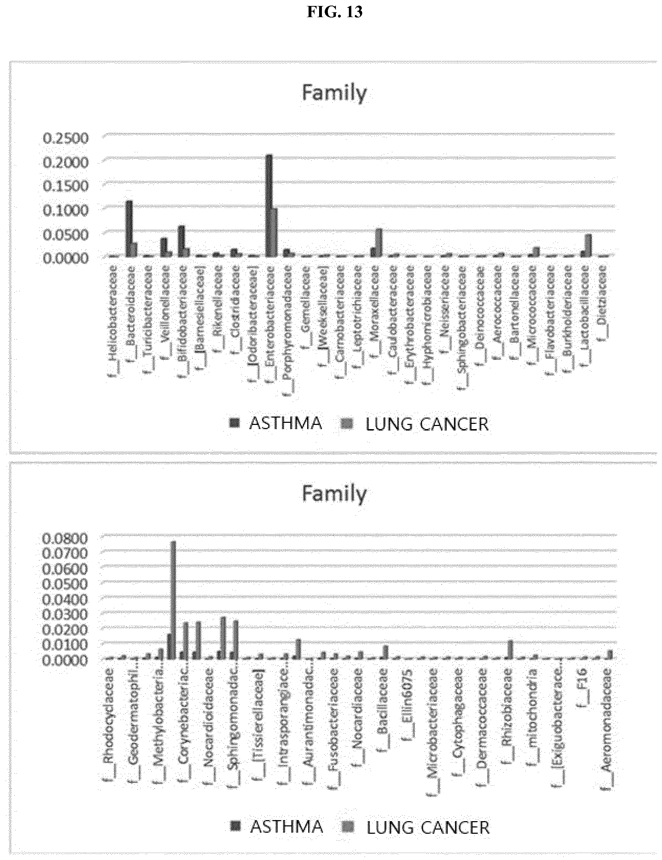

As a result of analyzing bacteria-derived extracellular vesicles in blood at a family level, a diagnostic model developed using, as a biomarker, one or more bacteria selected from the family *Helicobacteraceae,* the family *Bacteroidaceae,* the family *Turicibacteraceae,* the family *Veillonellaceae,* the family *Bijidobacteriaceae,* the family *Barnesiellaceae,* the family *Rikenellaceae,* the family *Clostridiaceae,* the family *Odoribacteraceae,* the family *Enterobacteriaceae,* the family *Porphyromonadaceae,* the family *Gemellaceae,* the family *Weeksellaceae,* the family *Carnobacteriaceae,* the family *Leptotrichiaceae,* the family *Moraxellaceae,* the family *Caulobacteraceae,* the family *Erythrobacteraceae,* the family *Hyphomicrobiaceae,* the family *Neisseriaceae,* the family *Sphingobacteriaceae,* the family *Deinococcaceae,* the family *Aerococcaceae,* the family *Bartonellaceae,* the family *Micrococcaceae,* the family *Flavobacteriaceae,* the family *Burkholderiaceae,* the family *Lactobacillaceae,* the family *Dietziaceae,* the family *Rhodocyclaceae,* the family *Xanthomonadaceae,* the family *Geodermatophilaceae,* the family *Actinomycetaceae,* the family *Methylobacteriaceae,* the family *Pseudomonadaceae,* the family *Corynebacteriaceae,* the family *Staphylococcaceae,* the family *Nocardioidaceae,* the family *Verrucomicrobiaceae,* the family *Sphingomonadaceae,* the family *Mycobacteriaceae,* the family *Tissierellaceae,* the family *Chitinophagaceae,* the family *Intrasporangiaceae,* the family *Propionibacteriaceae,* the family *Aurantimonadaceae,* the family *Planococcaceae,* the family *Fusobacteriaceae,* the family *Bradyrhizobiaceae,* the family *Nocardiaceae,* the family *Dermabacteraceae,* the family *Bacillaceae,* the family *Thermaceae,* the family Ellin6075, the family *Brevibacteriaceae,* the family *Microbacteriaceae,* the family *Rhodospirillaceae,* the family *Cytophagaceae,* the family *Fimbriimonadaceae,* the family *Dermacoccaceae,* the family *Chromatiaceae,* the family *Rhizobiaceae,* the family *Gordonžaceae,* the family *mitochondria,* the family *Pseudonocardiaceae,* the family *Exiguobacteraceae,* the family *Shewanellaceae,* the family F16, the family Rs-045, and the family *Aeromonadaceae* exhibited significant diagnostic performance for lung cancer (see Table 13 and FIG. 13).

**[Table 13]**

| | Asthma | | Lung Cancer | | t-test | | Training Set | | | Test Set | | |
|---|---|---|---|---|---|---|---|---|---|---|---|---|
| Taxon | Mea n | SD | Mea n | SD | p-val ue | fold | AU C | sensi tivit y | speci ficit y | AU C | sensi tivit y | sp ec ifi cit y |
| f_Helicobact eraceae | 0.00 05 | 0.00 22 | 0.00 00 | 0.00 02 | 0.00 24 | 0.05 | 0.79 | 0.64 | 0.86 | 0.77 | 0.68 | 0. 75 |
| f_Bacteroida ceae | 0.11 40 | 0.04 69 | 0.02 76 | 0.02 12 | 0.00 00 | 0.24 | 0.97 | 0.89 | 0.95 | 0.96 | 0.91 | 0. 91 |
| f_Turicibacte raceae | 0.00 17 | 0.00 53 | 0.00 04 | 0.00 17 | 0.00 09 | 0.25 | 0.80 | 0.62 | 0.86 | 0.77 | 0.65 | 0. 74 |
| f_Veillonella ceae | 0.03 75 | 0.02 88 | 0.00 94 | 0.00 93 | 0.00 00 | 0.25 | 0.94 | 0.79 | 0.91 | 0.93 | 0.82 | 0. 88 |
| f_Bifidobact eriaceae | 0.06 27 | 0.03 35 | 0.01 64 | 0.01 44 | 0.00 00 | 0.26 | 0.95 | 0.85 | 0.95 | 0.92 | 0.82 | 0. 88 |
| f_[Barnesiell aceae] | 0.00 24 | 0.00 50 | 0.00 08 | 0.00 25 | 0.00 00 | 0.32 | 0.80 | 0.64 | 0.85 | 0.79 | 0.65 | 0. 77 |
| f_Rikenellac eae | 0.00 70 | 0.00 87 | 0.00 25 | 0.00 50 | 0.00 00 | 0.36 | 0.83 | 0.68 | 0.84 | 0.81 | 0.68 | 0. 79 |
| f_Clostridiac eae | 0.01 51 | 0.01 43 | 0.00 59 | 0.00 78 | 0.00 00 | 0.39 | 0.82 | 0.66 | 0.86 | 0.81 | 0.69 | 0. 82 |
| f_[Odoribact eraceae] | 0.00 21 | 0.00 29 | 0.00 09 | 0.00 22 | 0.00 00 | 0.43 | 0.80 | 0.67 | 0.86 | 0.78 | 0.65 | 0. 75 |
| f_Enterobact eriaceae | 0.20 91 | 0.08 78 | 0.09 83 | 0.06 29 | 0.00 00 | 0.47 | 0.91 | 0.78 | 0.91 | 0.91 | 0.81 | 0. 85 |
| f_Porphyrom onadaceae | 0.01 45 | 0.00 90 | 0.00 69 | 0.01 05 | 0.00 00 | 0.47 | 0.85 | 0.68 | 0.85 | 0.83 | 0.72 | 0. 80 |
| f_Gemellace ae | 0.00 04 | 0.00 32 | 0.00 12 | 0.00 32 | 0.00 93 | 2.65 | 0.79 | 0.62 | 0.86 | 0.76 | 0.66 | 0. 75 |
| f_[Weeksella ceae] | 0.00 13 | 0.00 61 | 0.00 36 | 0.00 72 | 0.00 01 | 2.68 | 0.82 | 0.61 | 0.89 | 0.78 | 0.66 | 0. 80 |
| f_Carnobacte riaceae | 0.00 03 | 0.00 12 | 0.00 10 | 0.00 29 | 0.00 02 | 3.14 | 0.79 | 0.62 | 0.86 | 0.76 | 0.66 | 0. 77 |
| f_Leptotrichi aceae | 0.00 04 | 0.00 11 | 0.00 12 | 0.00 29 | 0.00 00 | 3.23 | 0.81 | 0.62 | 0.89 | 0.77 | 0.62 | 0. 79 |
| f_Moraxellac eae | 0.01 75 | 0.04 51 | 0.05 67 | 0.04 81 | 0.00 00 | 3.25 | 0.90 | 0.68 | 0.89 | 0.86 | 0.74 | 0. 82 |
| f_Caulobacte raceae | 0.00 15 | 0.00 30 | 0.00 50 | 0.00 72 | 0.00 00 | 3.31 | 0.85 | 0.61 | 0.89 | 0.80 | 0.71 | 0. 77 |
| f_Erythrobac teraceae | 0.00 03 | 0.00 28 | 0.00 10 | 0.00 45 | 0.02 35 | 3.36 | 0.79 | 0.62 | 0.86 | 0.75 | 0.66 | 0. 74 |
| f_Hyphomicr obiaceae | 0.00 01 | 0.00 06 | 0.00 04 | 0.00 15 | 0.00 20 | 3.36 | 0.79 | 0.62 | 0.87 | 0.76 | 0.65 | 0. 74 |
| f_Neisseriace ae | 0.00 16 | 0.00 64 | 0.00 58 | 0.01 19 | 0.00 00 | 3.61 | 0.82 | 0.62 | 0.87 | 0.80 | 0.69 | 0. 76 |
| f_Sphingoba cteriaceae | 0.00 03 | 0.00 10 | 0.00 13 | 0.00 41 | 0.00 00 | 3.91 | 0.80 | 0.61 | 0.86 | 0.76 | 0.65 | 0. 74 |
| | | | | | | | | | | | | |
| f_Deinococc aceae | 0.00 03 | 0.00 13 | 0.00 14 | 0.00 53 | 0.00 12 | 4.00 | 0.79 | 0.62 | 0.86 | 0.76 | 0.66 | 0. 75 |
| f_Aerococca ceae | 0.00 17 | 0.00 42 | 0.00 67 | 0.01 07 | 0.00 00 | 4.02 | 0.84 | 0.63 | 0.87 | 0.83 | 0.72 | 0. 79 |
| f_Bartonellac eae | 0.00 02 | 0.00 07 | 0.00 09 | 0.00 33 | 0.00 04 | 4.06 | 0.80 | 0.62 | 0.87 | 0.76 | 0.65 | 0. 74 |
| f_Micrococc aceae | 0.00 45 | 0.01 43 | 0.01 83 | 0.01 72 | 0.00 00 | 4.07 | 0.91 | 0.65 | 0.89 | 0.87 | 0.76 | 0. 79 |
| f_Flavobacte riaceae | 0.00 05 | 0.00 22 | 0.00 19 | 0.00 49 | 0.00 00 | 4.15 | 0.83 | 0.61 | 0.87 | 0.78 | 0.68 | 0. 77 |
| f_Burkholder iaceae | 0.00 04 | 0.00 10 | 0.00 17 | 0.00 64 | 0.00 05 | 4.16 | 0.79 | 0.62 | 0.86 | 0.77 | 0.66 | 0. 76 |
| f_Lactobacill aceae | 0.01 08 | 0.01 41 | 0.04 54 | 0.04 54 | 0.00 00 | 4.21 | 0.91 | 0.80 | 0.86 | 0.89 | 0.85 | 0. 82 |
| f_Dietziacea e | 0.00 03 | 0.00 08 | 0.00 11 | 0.00 49 | 0.00 31 | 4.25 | 0.79 | 0.62 | 0.87 | 0.76 | 0.66 | 0. 76 |
| f_Rhodocycl aceae | 0.00 03 | 0.00 09 | 0.00 12 | 0.00 45 | 0.00 06 | 4.31 | 0.80 | 0.62 | 0.86 | 0.75 | 0.65 | 0. 75 |
| f_Xanthomo nadaceae | 0.00 05 | 0.00 12 | 0.00 23 | 0.00 58 | 0.00 00 | 4.36 | 0.81 | 0.62 | 0.88 | 0.77 | 0.66 | 0. 76 |
| f_Geodermat ophilaceae | 0.00 03 | 0.00 12 | 0.00 12 | 0.00 34 | 0.00 00 | 4.44 | 0.80 | 0.61 | 0.86 | 0.79 | 0.65 | 0. 77 |
| f_Actinomyc etaceae | 0.00 08 | 0.00 38 | 0.00 35 | 0.00 60 | 0.00 00 | 4.46 | 0.83 | 0.62 | 0.89 | 0.82 | 0.65 | 0. 79 |
| f_Methyloba cteriaceae | 0.00 15 | 0.00 34 | 0.00 66 | 0.00 83 | 0.00 00 | 4.49 | 0.86 | 0.64 | 0.86 | 0.81 | 0.69 | 0. 74 |
| f_Pseudomo nadaceae | 0.01 60 | 0.01 54 | 0.07 65 | 0.05 31 | 0.00 00 | 4.77 | 0.97 | 0.91 | 0.95 | 0.95 | 0.93 | 0. 88 |
| f_Corynebact eriaceae | 0.00 48 | 0.00 55 | 0.02 38 | 0.02 41 | 0.00 00 | 4.95 | 0.91 | 0.79 | 0.85 | 0.90 | 0.84 | 0. 80 |
| f_Staphyloco ccaceae | 0.00 47 | 0.00 68 | 0.02 42 | 0.04 37 | 0.00 00 | 5.15 | 0.90 | 0.73 | 0.87 | 0.90 | 0.75 | 0. 84 |
| f_Nocardioid aceae | 0.00 03 | 0.00 08 | 0.00 17 | 0.00 46 | 0.00 00 | 5.16 | 0.81 | 0.61 | 0.85 | 0.77 | 0.68 | 0. 74 |
| f_Verrucomi crobiaceae | 0.00 52 | 0.00 60 | 0.02 74 | 0.02 35 | 0.00 00 | 5.29 | 0.91 | 0.79 | 0.83 | 0.87 | 0.76 | 0. 77 |
| f_Sphingomo nadaceae | 0.00 46 | 0.00 70 | 0.02 49 | 0.02 45 | 0.00 00 | 5.39 | 0.91 | 0.74 | 0.87 | 0.91 | 0.81 | 0. 82 |
| f_Mycobacte riaceae | 0.00 02 | 0.00 09 | 0.00 12 | 0.00 54 | 0.00 23 | 5.60 | 0.79 | 0.62 | 0.87 | 0.77 | 0.63 | 0. 76 |
| f_[Tissierella ceae] | 0.00 06 | 0.00 16 | 0.00 33 | 0.00 69 | 0.00 00 | 5.62 | 0.83 | 0.60 | 0.88 | 0.80 | 0.65 | 0. 82 |
| f_Chitinopha gaceae | 0.00 02 | 0.00 06 | 0.00 10 | 0.00 34 | 0.00 00 | 5.63 | 0.81 | 0.63 | 0.88 | 0.76 | 0.63 | 0. 73 |
| f_Intrasporan giaceae | 0.00 06 | 0.00 13 | 0.00 34 | 0.00 65 | 0.00 00 | 5.74 | 0.84 | 0.62 | 0.86 | 0.80 | 0.71 | 0. 76 |
| f_Propioniba cteriaceae | 0.00 21 | 0.00 31 | 0.01 26 | 0.01 17 | 0.00 00 | 5.85 | 0.91 | 0.75 | 0.87 | 0.93 | 0.79 | 0. 86 |
| f_Aurantimo nadaceae | 0.00 01 | 0.00 03 | 0.00 05 | 0.00 20 | 0.00 16 | 6.06 | 0.79 | 0.62 | 0.86 | 0.76 | 0.66 | 0. 74 |
| f_Planococca ceae | 0.00 07 | 0.00 13 | 0.00 45 | 0.01 08 | 0.00 00 | 6.20 | 0.87 | 0.64 | 0.85 | 0.83 | 0.66 | 0. 83 |
| f_Fusobacter iaceae | 0.00 06 | 0.00 26 | 0.00 35 | 0.00 90 | 0.00 00 | 6.21 | 0.83 | 0.62 | 0.88 | 0.83 | 0.68 | 0. 85 |
| f_Bradyrhizo biaceae | 0.00 03 | 0.00 08 | 0.00 20 | 0.00 54 | 0.00 00 | 6.25 | 0.82 | 0.60 | 0.86 | 0.77 | 0.65 | 0. 77 |
| f_Nocardiace ae | 0.00 08 | 0.00 19 | 0.00 48 | 0.01 03 | 0.00 00 | 6.32 | 0.86 | 0.65 | 0.87 | 0.82 | 0.74 | 0. 82 |
| f_Dermabact eraceae | 0.00 02 | 0.00 05 | 0.00 10 | 0.00 30 | 0.00 00 | 6.38 | 0.80 | 0.61 | 0.88 | 0.77 | 0.65 | 0. 76 |
| f_Bacillaceae | 0.00 13 | 0.00 20 | 0.00 85 | 0.01 49 | 0.00 00 | 6.44 | 0.88 | 0.64 | 0.88 | 0.82 | 0.72 | 0. 75 |
| f_Thermacea e | 0.00 02 | 0.00 14 | 0.00 17 | 0.00 39 | 0.00 00 | 7.32 | 0.83 | 0.60 | 0.89 | 0.79 | 0.66 | 0. 79 |
| f_Ellin6075 | 0.00 01 | 0.00 04 | 0.00 05 | 0.00 18 | 0.00 01 | 7.54 | 0.80 | 0.62 | 0.89 | 0.76 | 0.65 | 0. 77 |
| f_Brevibacte riaceae | 0.00 02 | 0.00 06 | 0.00 15 | 0.00 61 | 0.00 02 | 7.65 | 0.81 | 0.62 | 0.89 | 0.77 | 0.66 | 0. 76 |
| f_Microbacte riaceae | 0.00 02 | 0.00 08 | 0.00 13 | 0.00 38 | 0.00 00 | 7.69 | 0.82 | 0.62 | 0.86 | 0.79 | 0.68 | 0. 77 |
| | | | | | | | | | | | | |
| f_Rhodospiri llaceae | 0.00 02 | 0.00 11 | 0.00 15 | 0.00 62 | 0.00 02 | 7.94 | 0.79 | 0.62 | 0.87 | 0.77 | 0.66 | 0. 76 |
| f_Cytophaga ceae | 0.00 02 | 0.00 05 | 0.00 13 | 0.00 40 | 0.00 00 | 8.57 | 0.82 | 0.61 | 0.87 | 0.77 | 0.63 | 0. 78 |
| f_[Fimbriimo nadaceae] | 0.00 01 | 0.00 04 | 0.00 09 | 0.00 51 | 0.00 48 | 8.78 | 0.81 | 0.62 | 0.87 | 0.76 | 0.65 | 0. 78 |
| f_Dermacocc aceae | 0.00 02 | 0.00 05 | 0.00 19 | 0.00 48 | 0.00 00 | 9.05 | 0.83 | 0.63 | 0.88 | 0.80 | 0.69 | 0. 78 |
| f_[Chromatia ceae] | 0.00 01 | 0.00 06 | 0.00 11 | 0.00 62 | 0.00 56 | 9.57 | 0.80 | 0.62 | 0.87 | 0.75 | 0.66 | 0. 74 |
| f_Rhizobiace ae | 0.00 12 | 0.00 25 | 0.01 18 | 0.01 50 | 0.00 00 | 9.97 | 0.92 | 0.76 | 0.88 | 0.90 | 0.76 | 0. 83 |
| f_Gordoniac eae | 0.00 01 | 0.00 05 | 0.00 13 | 0.00 48 | 0.00 00 | 10.20 | 0.80 | 0.62 | 0.85 | 0.77 | 0.65 | 0. 76 |
| f_mitochondr ia | 0.00 03 | 0.00 11 | 0.00 27 | 0.00 81 | 0.00 00 | 10.41 | 0.84 | 0.60 | 0.89 | 0.75 | 0.60 | 0. 74 |
| f_Pseudonoc ardiaceae | 0.00 01 | 0.00 02 | 0.00 06 | 0.00 25 | 0.00 01 | 11.72 | 0.80 | 0.63 | 0.86 | 0.76 | 0.63 | 0. 75 |
| f_[Exiguobac teraceae] | 0.00 00 | 0.00 02 | 0.00 06 | 0.00 33 | 0.00 48 | 23.39 | 0.80 | 0.62 | 0.86 | 0.77 | 0.65 | 0. 79 |
| f_Shewanella ceae | 0.00 00 | 0.00 04 | 0.00 09 | 0.00 40 | 0.00 03 | 24.54 | 0.80 | 0.62 | 0.86 | 0.77 | 0.66 | 0. 77 |
| f_F16 | 0.00 01 | 0.00 05 | 0.00 16 | 0.00 40 | 0.00 00 | 24.68 | 0.85 | 0.63 | 0.88 | 0.78 | 0.68 | 0. 74 |
| f_Rs-045 | 0.00 01 | 0.00 05 | 0.00 17 | 0.00 58 | 0.00 00 | 26.38 | 0.83 | 0.62 | 0.90 | 0.78 | 0.65 | 0. 78 |
| f_Aeromona daceae | 0.00 02 | 0.00 07 | 0.00 56 | 0.03 20 | 0.00 30 | 34.42 | 0.82 | 0.62 | 0.87 | 0.78 | 0.68 | 0. 74 |

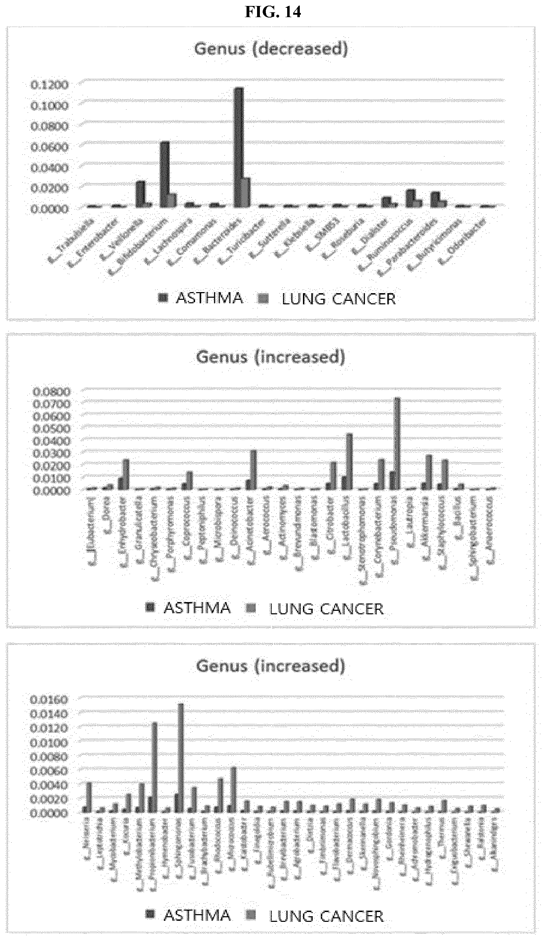

As a result of analyzing bacteria-derived extracellular vesicles in blood at a genus level, a diagnostic model developed using, as a biomarker, one or more bacteria selected from of the genus *Trabulsiella,* the genus *Enterobacter,* the genus *Veillonella,* the genus *Bifidobacterium,* the genus *Lachnospira,* the genus *Comamonas,* the genus *Bacteroides,* the genus *Turicibacter,* the genus *Sutterella,* the genus *Klebsiella,* the genus SMB53, the genus *Roseburia,* the genus *Dialister,* the genus *Ruminococcus,* the genus *Parabacteroides,* the genus *Butyricimonas,* the genus *Odoribacter,* the genus *Eubacterium,* the genus *Dorea,* the genus *Enhydrobacter,* the genus *Granulicatella,* the genus *Chryseobacterium,* the genus *Porphyromonas,* the genus *Coprococcus,* the genus *Peptoniphilus,* the genus *Microbispora,* the genus *Deinococcus,* the genus *Acinetobacter,* the genus *Aerococcus,* the genus *Actinomyces,* the genus *Brevundimonas,* the genus *Blastomonas,* the genus *Citrobacter,* the genus *Lactobacillus,* the genus *Stenotrophomonas,* the genus *Corynebacterium,* the genus *Pseudomonas,* the genus *Lautropia,* the genus *Akkermansia,* the genus *Staphylococcus,* the genus *Bacillus,* the genus *Sphingobacterium,* the genus *Anaerococcus,* the genus *Neisseria,* the genus *Leptotrichia,* the genus *Mycobacterium,* the genus *Kocuria,* the genus *Methylobacterium,* the genus *Propionibacterium,* the genus *Hymenobacter,* the genus *Sphingomonas,* the genus *Fusobacterium,* the genus *Brachybacterium,* the genus *Rhodococcus,* the genus *Micrococcus,* the genus *Kaistobacter,* the genus *Finegoldia,* the genus *Rubellimicrobium,* the genus *Brevibacterium,* the genus *Agrobacterium,* the genus *Dietzia,* the genus *Fimbriimonas,* the genus *Flavobacterium,* the genus *Dermacoccus,* the genus *Skermanella,* the genus *Novosphingobium,* the genus *Gordonia,* the genus *Rheinheimera,* the genus *Achromobacter,* the genus *Hydrogenophilus,* the genus *Thermus,* the genus *Exiguobacterium,* the genus *Shewanella,* the genus *Ralstonia,* and the genus *Alkanindiges* exhibited significant diagnostic performance for lung cancer (see Table 14 and FIG. 14).

**[Table 14]**

| | Asthma | | Lung Cancer | | t-test | | Training Set | | | Test Set | | |
|---|---|---|---|---|---|---|---|---|---|---|---|---|
| Taxon | Mea n | SD | Mea n | SD | p-val ue | fold | AU C | sensi tivity | speci ficity | AUC | sensit ivity | spe cifi city |
| g_Trabulsie lla | 0.00 07 | 0.00 12 | 0.00 00 | 0.00 02 | 0.00 00 | 0.04 | 0.92 | 0.75 | 0.92 | 0.91 | 0.82 | 0.8 9 |
| g_Enteroba cter | 0.00 16 | 0.00 13 | 0.00 02 | 0.00 18 | 0.00 00 | 0.11 | 0.93 | 0.77 | 0.93 | 0.91 | 0.79 | 0.8 7 |
| g_Veillonel la | 0.02 43 | 0.02 48 | 0.00 35 | 0.00 54 | 0.00 00 | 0.14 | 0.94 | 0.82 | 0.91 | 0.93 | 0.84 | 0.8 7 |
| g_Bifidoba cterium | 0.06 23 | 0.03 36 | 0.01 23 | 0.01 20 | 0.00 00 | 0.20 | 0.96 | 0.87 | 0.96 | 0.95 | 0.82 | 0.9 2 |
| g_Lachnos pira | 0.00 37 | 0.01 30 | 0.00 08 | 0.00 22 | 0.00 13 | 0.23 | 0.86 | 0.67 | 0.87 | 0.82 | 0.72 | 0.7 8 |
| g_Comamo nas | 0.00 31 | 0.00 91 | 0.00 07 | 0.00 23 | 0.00 02 | 0.23 | 0.81 | 0.63 | 0.86 | 0.80 | 0.69 | 0.7 8 |
| g_Bacteroi des | 0.11 40 | 0.04 69 | 0.02 76 | 0.02 12 | 0.00 00 | 0.24 | 0.97 | 0.89 | 0.95 | 0.96 | 0.91 | 0.9 1 |
| g_Turiciba cter | 0.00 17 | 0.00 53 | 0.00 04 | 0.00 17 | 0.00 09 | 0.25 | 0.80 | 0.62 | 0.86 | 0.77 | 0.65 | 0.7 4 |
| g_Sutterell a | 0.00 14 | 0.00 29 | 0.00 04 | 0.00 13 | 0.00 00 | 0.27 | 0.80 | 0.66 | 0.84 | 0.77 | 0.62 | 0.7 7 |
| g_Klebsiell a | 0.00 18 | 0.00 12 | 0.00 06 | 0.00 20 | 0.00 00 | 0.31 | 0.90 | 0.72 | 0.87 | 0.88 | 0.79 | 0.7 9 |
| g_SMB53 | 0.00 23 | 0.00 33 | 0.00 07 | 0.00 15 | 0.00 00 | 0.32 | 0.81 | 0.63 | 0.86 | 0.78 | 0.69 | 0.7 4 |
| g_Roseburi a | 0.00 21 | 0.00 29 | 0.00 07 | 0.00 31 | 0.00 00 | 0.33 | 0.80 | 0.64 | 0.86 | 0.78 | 0.65 | 0.7 6 |
| g_Dialister | 0.00 90 | 0.00 77 | 0.00 33 | 0.00 47 | 0.00 00 | 0.37 | 0.86 | 0.67 | 0.87 | 0.86 | 0.76 | 0.7 8 |
| g_Ruminoc | 0.01 | 0.01 | 0.00 | 0.01 | 0.00 | 0.38 | 0.89 | 0.72 | 0.88 | 0.84 | 0.76 | 0.7 |
| occus | 63 | 12 | 62 | 02 | 00 | | | | | | | 8 |
| g_Parabact eroides | 0.01 38 | 0.00 86 | 0.00 55 | 0.00 99 | 0.00 00 | 0.40 | 0.87 | 0.70 | 0.87 | 0.86 | 0.75 | 0.8 6 |
| g_Butyrici monas | 0.00 12 | 0.00 19 | 0.00 05 | 0.00 16 | 0.00 00 | 0.42 | 0.80 | 0.64 | 0.85 | 0.78 | 0.63 | 0.7 4 |
| g_Odoribac ter | 0.00 09 | 0.00 17 | 0.00 04 | 0.00 15 | 0.00 08 | 0.46 | 0.80 | 0.64 | 0.87 | 0.76 | 0.65 | 0.7 6 |
| g_[Eubacte rium] | 0.00 07 | 0.00 13 | 0.00 15 | 0.00 28 | 0.00 00 | 2.09 | 0.81 | 0.61 | 0.87 | 0.76 | 0.63 | 0.7 6 |
| g_Dorea | 0.00 17 | 0.00 72 | 0.00 37 | 0.00 61 | 0.00 09 | 2.18 | 0.82 | 0.61 | 0.87 | 0.79 | 0.69 | 0.7 8 |
| g_Enhydro bacter | 0.00 93 | 0.04 25 | 0.02 36 | 0.02 93 | 0.00 00 | 2.54 | 0.84 | 0.62 | 0.86 | 0.78 | 0.68 | 0.7 5 |
| g_Granulic atella | 0.00 03 | 0.00 11 | 0.00 07 | 0.00 24 | 0.00 51 | 2.58 | 0.79 | 0.62 | 0.86 | 0.75 | 0.66 | 0.7 6 |
| g_Chryseo bacterium | 0.00 07 | 0.00 25 | 0.00 19 | 0.00 42 | 0.00 00 | 2.74 | 0.80 | 0.62 | 0.88 | 0.77 | 0.66 | 0.7 6 |
| g_Porphyro monas | 0.00 05 | 0.00 36 | 0.00 13 | 0.00 33 | 0.00 65 | 2.78 | 0.79 | 0.62 | 0.86 | 0.76 | 0.66 | 0.7 5 |
| g_Coproco ccus | 0.00 47 | 0.00 50 | 0.01 41 | 0.01 46 | 0.00 00 | 2.97 | 0.85 | 0.64 | 0.89 | 0.82 | 0.71 | 0.7 9 |
| g_Peptonip hilus | 0.00 01 | 0.00 05 | 0.00 04 | 0.00 13 | 0.00 12 | 3.17 | 0.79 | 0.61 | 0.86 | 0.76 | 0.66 | 0.7 6 |
| g_Microbis pora | 0.00 02 | 0.00 06 | 0.00 06 | 0.00 24 | 0.00 42 | 3.57 | 0.79 | 0.62 | 0.86 | 0.75 | 0.65 | 0.7 5 |
| g_Deinoco ccus | 0.00 03 | 0.00 13 | 0.00 13 | 0.00 52 | 0.00 20 | 3.81 | 0.79 | 0.62 | 0.86 | 0.76 | 0.66 | 0.7 5 |
| g_Acinetob acter | 0.00 76 | 0.01 28 | 0.03 10 | 0.02 78 | 0.00 00 | 4.09 | 0.91 | 0.78 | 0.84 | 0.89 | 0.78 | 0.8 5 |
| g_Aerococ cus | 0.00 05 | 0.00 20 | 0.00 22 | 0.00 75 | 0.00 02 | 4.10 | 0.79 | 0.62 | 0.86 | 0.77 | 0.66 | 0.7 7 |
| g_Actinom yces | 0.00 08 | 0.00 38 | 0.00 32 | 0.00 57 | 0.00 00 | 4.12 | 0.82 | 0.62 | 0.89 | 0.81 | 0.65 | 0.7 8 |
| g_Brevundi | 0.00 03 | 0.00 08 | 0.00 12 | 0.00 34 | 0.00 00 | 4.24 | 0.80 | 0.61 | 0.87 | 0.78 | 0.66 | 0.7 6 |
| g_Blastom onas | 0.00 02 | 0.00 16 | 0.00 07 | 0.00 24 | 0.00 22 | 4.33 | 0.80 | 0.62 | 0.86 | 0.77 | 0.66 | 0.7 6 |
| g_Citrobact er | 0.00 50 | 0.00 41 | 0.02 18 | 0.02 20 | 0.00 00 | 4.34 | 0.87 | 0.65 | 0.87 | 0.85 | 0.75 | 0.7 9 |
| g_Lactobac illus | 0.01 01 | 0.01 40 | 0.04 44 | 0.04 54 | 0.00 00 | 4.39 | 0.91 | 0.79 | 0.85 | 0.89 | 0.85 | 0.7 9 |
| g_Stenotro phomonas | 0.00 01 | 0.00 05 | 0.00 05 | 0.00 19 | 0.00 05 | 4.58 | 0.79 | 0.62 | 0.86 | 0.75 | 0.65 | 0.7 7 |
| g_Coryneb acterium | 0.00 48 | 0.00 55 | 0.02 38 | 0.02 41 | 0.00 00 | 4.95 | 0.91 | 0.79 | 0.85 | 0.90 | 0.84 | 0.8 0 |
| g_Pseudom onas | 0.01 44 | 0.01 45 | 0.07 34 | 0.05 06 | 0.00 00 | 5.11 | 0.97 | 0.91 | 0.95 | 0.96 | 0.93 | 0.8 8 |
| g_Lautropi a | 0.00 02 | 0.00 05 | 0.00 13 | 0.00 60 | 0.00 27 | 5.15 | 0.79 | 0.62 | 0.86 | 0.77 | 0.66 | 0.7 5 |
| g_Akkerma nsia | 0.00 52 | 0.00 60 | 0.02 73 | 0.02 36 | 0.00 00 | 5.27 | 0.91 | 0.78 | 0.82 | 0.87 | 0.78 | 0.7 7 |
| g_Staphylo coccus | 0.00 44 | 0.00 62 | 0.02 33 | 0.04 35 | 0.00 00 | 5.29 | 0.90 | 0.72 | 0.89 | 0.90 | 0.76 | 0.8 3 |
| g_Bacillus | 0.00 08 | 0.00 16 | 0.00 42 | 0.01 08 | 0.00 00 | 5.34 | 0.84 | 0.62 | 0.89 | 0.78 | 0.66 | 0.8 0 |
| g_Sphingo bacterium | 0.00 01 | 0.00 06 | 0.00 05 | 0.00 19 | 0.00 11 | 5.39 | 0.79 | 0.61 | 0.86 | 0.76 | 0.66 | 0.7 5 |
| g_Anaeroc occus | 0.00 03 | 0.00 13 | 0.00 14 | 0.00 41 | 0.00 00 | 5.39 | 0.80 | 0.62 | 0.86 | 0.77 | 0.63 | 0.7 8 |
| g_Neisseria | 0.00 08 | 0.00 26 | 0.00 41 | 0.01 02 | 0.00 00 | 5.46 | 0.83 | 0.62 | 0.87 | 0.80 | 0.68 | 0.7 8 |
| g_Leptotric hia | 0.00 01 | 0.00 05 | 0.00 06 | 0.00 21 | 0.00 00 | 5.54 | 0.80 | 0.62 | 0.87 | 0.76 | 0.65 | 0.7 8 |
| g_Mycobac terium | 0.00 02 | 0.00 09 | 0.00 12 | 0.00 54 | 0.00 23 | 5.60 | 0.79 | 0.62 | 0.87 | 0.77 | 0.63 | 0.7 6 |
| g_Kocuria | 0.00 04 | 0.00 14 | 0.00 25 | 0.00 58 | 0.00 00 | 5.66 | 0.83 | 0.62 | 0.88 | 0.80 | 0.68 | 0.8 2 |
| g_Methylo bacterium | 0.00 07 | 0.00 20 | 0.00 40 | 0.00 68 | 0.00 00 | 5.70 | 0.84 | 0.62 | 0.86 | 0.79 | 0.69 | 0.7 8 |
| g_Propioni bacterium | 0.00 21 | 0.00 31 | 0.01 25 | 0.01 17 | 0.00 00 | 5.84 | 0.91 | 0.75 | 0.87 | 0.93 | 0.79 | 0.8 5 |
| g_Hymeno bacter | 0.00 01 | 0.00 04 | 0.00 06 | 0.00 21 | 0.00 01 | 5.97 | 0.80 | 0.60 | 0.86 | 0.75 | 0.66 | 0.7 5 |
| g_Sphingo monas | 0.00 25 | 0.00 40 | 0.01 51 | 0.01 66 | 0.00 00 | 5.99 | 0.90 | 0.68 | 0.90 | 0.89 | 0.81 | 0.7 8 |
| g_Fusobact erium | 0.00 06 | 0.00 26 | 0.00 35 | 0.00 90 | 0.00 00 | 6.21 | 0.83 | 0.62 | 0.88 | 0.83 | 0.68 | 0.8 5 |
| g_Brachyb acterium | 0.00 01 | 0.00 05 | 0.00 09 | 0.00 30 | 0.00 00 | 6.22 | 0.80 | 0.62 | 0.87 | 0.76 | 0.66 | 0.7 5 |
| g_Rhodoco ccus | 0.00 08 | 0.00 19 | 0.00 47 | 0.01 02 | 0.00 00 | 6.25 | 0.86 | 0.65 | 0.87 | 0.81 | 0.74 | 0.8 0 |
| g_Microco ccus | 0.00 10 | 0.00 17 | 0.00 63 | 0.00 98 | 0.00 00 | 6.46 | 0.86 | 0.64 | 0.88 | 0.81 | 0.74 | 0.7 4 |
| g_Kaistoba cter | 0.00 02 | 0.00 10 | 0.00 16 | 0.00 55 | 0.00 00 | 6.46 | 0.81 | 0.61 | 0.88 | 0.76 | 0.65 | 0.7 4 |
| g_Finegold ia | 0.00 01 | 0.00 06 | 0.00 08 | 0.00 35 | 0.00 03 | 7.22 | 0.80 | 0.62 | 0.87 | 0.77 | 0.65 | 0.7 6 |
| g_Rubellim icrobium | 0.00 01 | 0.00 07 | 0.00 07 | 0.00 40 | 0.00 74 | 7.31 | 0.79 | 0.62 | 0.86 | 0.76 | 0.66 | 0.7 4 |
| g_Brevibac terium | 0.00 02 | 0.00 06 | 0.00 15 | 0.00 61 | 0.00 02 | 7.65 | 0.81 | 0.62 | 0.89 | 0.77 | 0.66 | 0.7 6 |
| g_Agrobact erium | 0.00 02 | 0.00 07 | 0.00 15 | 0.00 36 | 0.00 00 | 7.73 | 0.82 | 0.63 | 0.88 | 0.79 | 0.65 | 0.7 8 |
| g_Dietzia | 0.00 01 | 0.00 06 | 0.00 10 | 0.00 48 | 0.00 17 | 8.13 | 0.80 | 0.63 | 0.87 | 0.76 | 0.66 | 0.7 6 |
| g_Fimbrii monas | 0.00 01 | 0.00 04 | 0.00 09 | 0.00 50 | 0.00 60 | 8.88 | 0.81 | 0.62 | 0.87 | 0.76 | 0.65 | 0.7 8 |
| g_Flavobac terium | 0.00 01 | 0.00 05 | 0.00 12 | 0.00 40 | 0.00 00 | 8.98 | 0.82 | 0.61 | 0.86 | 0.77 | 0.68 | 0.7 6 |
| g_Dermaco ccus | 0.00 02 | 0.00 05 | 0.00 19 | 0.00 48 | 0.00 00 | 9.05 | 0.83 | 0.63 | 0.88 | 0.80 | 0.69 | 0.7 8 |
| g_Skerman ella | 0.00 01 | 0.00 09 | 0.00 12 | 0.00 58 | 0.00 20 | 9.54 | 0.79 | 0.62 | 0.87 | 0.77 | 0.66 | 0.7 6 |
| g_Novosph ingobium | 0.00 02 | 0.00 09 | 0.00 19 | 0.00 43 | 0.00 00 | 9.63 | 0.83 | 0.62 | 0.86 | 0.80 | 0.66 | 0.7 9 |
| g_Gordoni a | 0.00 01 | 0.00 05 | 0.00 13 | 0.00 48 | 0.00 00 | 10.2 0 | 0.80 | 0.62 | 0.85 | 0.77 | 0.65 | 0.7 6 |
| g_Rheinhei mera | 0.00 01 | 0.00 05 | 0.00 10 | 0.00 62 | 0.00 75 | 11.5 1 | 0.79 | 0.63 | 0.87 | 0.76 | 0.66 | 0.7 4 |
| g_Achrom obacter | 0.00 01 | 0.00 04 | 0.00 06 | 0.00 28 | 0.00 03 . | 12.3 9 | 0.81 | 0.62 | 0.88 | 0.75 | 0.63 | 0.7 6 |
| g_Hydroge nophilus | 0.00 01 | 0.00 07 | 0.00 09 | 0.00 41 | 0.00 11 | 12.5 4 | 0.79 | 0.62 | 0.86 | 0.76 | 0.66 | 0.7 5 |
| g_Thermus | 0.00 01 | 0.00 07 | 0.00 17 | 0.00 38 | 0.00 00 | 16.8 2 | 0.83 | 0.61 | 0.88 | 0.80 | 0.66 | 0.7 9 |
| g_Exiguob acterium | 0.00 00 | 0.00 02 | 0.00 06 | 0.00 33 | 0.00 49 | 23.7 5 | 0.80 | 0.62 | 0.86 | 0.77 | 0.65 | 0.7 9 |
| g_Shewane lla | 0.00 00 | 0.00 04 | 0.00 09 | 0.00 40 | 0.00 03 | 24.6 1 | 0.80 | 0.62 | 0.86 | 0.77 | 0.66 | 0.7 7 |
| g_Ralstoni a | 0.00 00 | 0.00 01 | 0.00 10 | 0.00 33 | 0.00 00 | 28.3 1 | 0.82 | 0.62 | 0.88 | 0.80 | 0.66 | 0.7 9 |
| g_Alkanind iges | 0.00 00 | 0.00 01 | 0.00 05 | 0.00 34 | 0.00 95 | 40.3 4 | 0.80 | 0.62 | 0.86 | 0.74 | 0.66 | 0.7 4 |

### [Industrial Applicability]

According to the present invention, a risk group for lung cancer can be diagnosed early and predicted by predicting a risk for lung cancer through metagenomic analysis of bacteria or bacteria-derived extracellular vesicles from a human body-derived sample, and thus the onset of lung cancer can be delayed or lung cancer may be prevented through appropriate management, and, even after lung cancer occurs, early diagnosis for lung cancer can be implemented, thereby lowering a disease rate and increasing therapeutic effects. In addition, patients diagnosed with lung cancer are able to avoid exposure to causative factors predicted by metagenomic analysis, whereby the progression of lung cancer is ameliorated, or recurrence of lung cancer can be prevented.

## Claims

1. A method of providing information for lung cancer diagnosis, the method comprising the following processes:
(a) extracting DNA from extracellular vesicles isolated from a subject sample;
(b) performing polymerase chain reaction (PCR) on the extracted DNA using a pair of primers having SEQ ID NO:1 and SEQ ID NO: 2; and
(c) making said diagnosis by, through sequencing of the PCR product, comparing an increase or decrease in content of bacteria-derived extracellular vesicles of the subject sample with that of
(i) a normal individual-derived sample, wherein the extracellular vesicles are derived from one or more bacteria selected from the group consisting of:
the phylum *Acidobacteria* and the phylum *Chloroflexi;* the class *Thermomicrobia* and the class *Solibacteres;*
the order *Turicibacterales,* the order *Rickettsiales,* the order *Alteromonadales,* the order RF32, the order *Thermales,* the order JG30-KF-CM45, the order 1025, the order *Solibacterales,* and the order *Aeromonadales;*
the family *Turicibacteraceae,* the family *Clostridiaceae,* the family S24-7, the family *Rhizobiaceae,* the family *mitochondria,* the family F16, the family *Gordoniaceae,* the family *Rhodospirillaceae,* the family *Thermaceae,* the family *Shewanellaceae,* the family Ellin6075, the family Rs-045, and the family *Aeromonadaceae;* and
the genus *Chromohalobacter,* the genus *Geobacillus,* the genus *Proteus,* the genus *Megamonas,* the genus *Moraxella,* the genus *Alloiococcus,* the genus *Turicibacter,* the genus SMB53, the genus *Veillonella,* the genus *Peptoniphilus,* the genus *Comamonas,* the genus *Hymenobacter,* the genus *Citrobacter,* the genus *Novosphingobium,* the genus *Gordonia,* the genus *Aerococcus,* the genus *Thermus,* the genus *Shewanella,* and the genus *Achromobacter;*
(ii) a chronic obstructive pulmonary disease patient-derived sample, wherein the extracellular vesicles are derived from one or more bacteria selected from the group consisting of:
the order *Bacillales,* the order *Rickettsiales,* and the order 1025;
the family *Nocardiaceae,* and the family Rs-045; and
the genus *Alloiococcus,* the genus *Moraxella,* the genus *Brevundimonas,* the genus *Enhydrobacter,* the genus *Comamonas,* and the genus *Rhodococcus;* OR
(iii) an asthma patient-derived sample wherein the extracellular vesicles are derived from one or more bacteria selected from the group consisting of:
the phylum *Bacteroidetes,* the phylum *Cyanobacteria,* the phylum TM7, the phylum *Fusobacteria,* the phylum *Thermi,* the phylum *Verrucomicrobia,* the phylum *Armatimonadetes,* the phylum *Acidobacteria,* the phylum *Gemmatimonadetes,* and the phylum *Chloroflexi;*
the class *Bacteroidia,* the class *Bacilli,* the class *Flavobacteriia,* the class *Sphingobacteriia,* the class *Alphaproteobacteria,* the class *Fusobacteriia,* the class TM7-3, the class *Deinococci,* the class *Verrucomicrobiae,* the class *Saprospirae,* the class *Chloroplast,* the class *Cytophagia,* the class *Fimbriimonadia,* the class *Chloracidobacteria,* the class *Thermomicrobia,* the class *Thermoleophilia,* and the class *Solibacteres;*
the order YS2, the order *Turicibacterales,* the order *Bifidobacteriales,* the order *Bacteroidales,* the order *Enterobacteriales,* the order *Rhodobacterales,* the order *Gemellales,* the order *Flavobacteriales,* the order *Caulobacterales,* the order *Neisseriales,* the order *Sphingobacteriales,* the order *Deinococcales,* the order *Pseudomonadales,* the order *Rhodocyclales,* the order *Xanthomonadales,* the order *Fusobacteriales,* the order *Actinomycetales,* the order *Sphingomonadales,* the order *Verrucomicrobiales,* the order *Saprospirales,* the order *Rhizobiales,* the order *Bacillales,* the order *Streptophyta,* the order *Cytophagales,* the order *Thermales,* the order *Fimbriimonadales,* the order CW040, the order *Rickettsiales,* the order RB41, the order *Alteromonadales,* the order JG30-KF-CM45, the order 1025, the order *Aeromonadales,* and the order *Solibacterales;*
the family *Helicobacteraceae,* the family *Bacteroidaceae,* the family *Turicibacteraceae,* the family *Veillonellaceae,* the family *Bifidobacteriaceae,* the family *Barnesiellaceae,* the family *Rikenellaceae,* the family *Clostridiaceae,* the family *Odoribacteraceae,* the family *Enterobacteriaceae,* the family *Porphyromonadaceae,* the family *Gemellaceae,* the family *Weeksellaceae,* the family *Carnobacteriaceae,* the family *Leptotrichiaceae,* the family *Moraxellaceae,* the family *Caulobacteraceae,* the family *Erythrobacteraceae,* the family *Hyphomicrobiaceae,* the family *Neisseriaceae,* the family *Sphingobacteriaceae,* the family *Deinococcaceae,* the family *Aerococcaceae,* the family *Bartonellaceae,* the family *Micrococcaceae,* the family *Flavobacteriaceae,* the family *Burkholderiaceae,* the family *Lactobacillaceae,* the family *Dietziaceae,* the family *Rhodocyclaceae,* the family *Xanthomonadaceae,* the family *Geodermatophilaceae,* the family *Actinomycetaceae,* the family *Methylobacteriaceae,* the family *Pseudomonadaceae,* the family *Corynebacteriaceae,* the family *Nocardioidaceae,* the family *Verrucomicrobiaceae,* the family *Sphingomonadaceae,* the family *Mycobacteriaceae,* the family *Tissierellaceae,* the family *Chitinophagaceae,* the family *Intrasporangiaceae,* the family *Propionibacteriaceae,* the family *Aurantimonadaceae,* the family *Planococcaceae,* the family *Fusobacteriaceae,* the family *Bradyrhizobiaceae,* the family *Nocardiaceae,* the family *Dermabacteraceae,* the family *Bacillaceae,* the family *Thermaceae,* the family Ellin6075, the family *Brevibacteriaceae,* the family *Microbacteriaceae,* the family *Rhodospirillaceae,* the family *Cytophagaceae,* the family *Fimbriimonadaceae,* the family *Dermacoccaceae,* the family *Chromatiaceae,* the family *Rhizobiaceae,* the family *Gordoniaceae,* the family *mitochondria,* the family *Pseudonocardiaceae,* the family *Exiguobacteraceae,* the family *Shewanellaceae,* the family F16, the family Rs-045, and the family *Aeromonadaceae;* and
the genus *Trabulsiella,* the genus *Veillonella,* the genus *Bifidobacterium,* the genus *Lachnospira,* the genus *Comamonas,* the genus *Bacteroides,* the genus *Turicibacter,* the genus *Sutterella,* the genus *Klebsiella,* the genus SMB53, the genus *Roseburia,* the genus *Dialister,* the genus *Ruminococcus,* the genus *Parabacteroides,* the genus *Butyricimonas,* the genus *Odoribacter,* the genus *Eubacterium,* the genus *Dorea,* the genus *Enhydrobacter,* the genus *Granulicatella,* the genus *Chryseobacterium,* the genus *Porphyromonas,* the genus *Coprococcus,* the genus *Peptoniphilus,* the genus *Microbispora,* the genus *Deinococcus,* the genus *Aerococcus,* the genus *Actinomyces,* the genus *Brevundimonas,* the genus *Blastomonas,* the genus *Citrobacter,* the genus *Lactobacillus,* the genus *Stenotrophomonas,* the genus *Corynebacterium,* the genus *Lautropia,* the genus *Akkermansia,* the genus *Bacillus,* the genus *Sphingobacterium,* the genus *Anaerococcus,* the genus *Neisseria,* the genus *Leptotrichia,* the genus *Mycobacterium,* the genus *Kocuria,* the genus *Methylobacterium,* the genus *Propionibacterium,* the genus *Hymenobacter,* the genus *Sphingomonas,* the genus *Fusobacterium,* the genus *Brachybacterium,* the genus *Rhodococcus,* the genus *Micrococcus,* the genus *Kaistobacter,* the genus *Finegoldia,* the genus *Rubellimicrobium,* the genus *Brevibacterium,* the genus *Agrobacterium,* the genus *Dietzia,* the genus *Fimbriimonas,* the genus *Flavobacterium,* the genus *Dermacoccus,* the genus *Skermanella,* the genus *Novosphingobium,* the genus *Gordonia,* the genus *Rheinheimera,* the genus *Achromobacter,* the genus *Hydrogenophilus,* the genus *Thermus,* the genus *Exiguobacterium,* the genus *Shewanella,* the genus *Ralstonia,* and the genus *Alkanindiges;*
and wherein the subject sample is blood.

2. A method of diagnosing lung cancer, the method comprising the following processes:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) making said diagnosis by, through sequencing of a product of the PCR, comparing an increase or decrease in content of bacteria-derived extracellular vesicles of the subject sample with that of
(i) a normal individual-derived sample, wherein the extracellular vesicles are derived from one or more bacteria selected from the group consisting of:
the phylum *Acidobacteria* and the phylum *Chloroflexi;* the class *Thermomicrobia* and the class *Solibacteres;*
the order *Turicibacterales,* the order *Rickettsiales,* the order *Alteromonadales,* the order RF32, the order *Thermales,* the order JG30-KF-CM45, the order 1025, the order *Solibacterales,* and the order *Aeromonadales;*
the family *Turicibacteraceae,* the family *Clostridiaceae,* the family S24-7, the family *Rhizobiaceae,* the family *mitochondria,* the family F16, the family *Gordoniaceae,* the family *Rhodospirillaceae,* the family *Thermaceae,* the family *Shewanellaceae,* the family Ellin6075, the family Rs-045, and the family *Aeromonadaceae;* and
the genus *Chromohalobacter,* the genus *Geobacillus,* the genus *Proteus,* the genus *Megamonas,* the genus *Moraxella,* the genus *Alloiococcus,* the genus *Turicibacter,* the genus SMB53, the genus *Veillonella,* the genus *Peptoniphilus,* the genus *Comamonas,* the genus *Hymenobacter,* the genus *Citrobacter,* the genus *Novosphingobium,* the genus *Gordonia,* the genus *Aerococcus,* the genus *Thermus,* the genus *Shewanella,* and the genus *Achromobacter;*
(ii) a chronic obstructive pulmonary disease patient-derived sample, wherein the extracellular vesicles are derived from one or more bacteria selected from the group consisting of:
the order *Bacillales,* the order *Rickettsiales,* and the order 1025;
the family *Nocardiaceae,* and the family Rs-045; and
the genus *Alloiococcus,* the genus *Moraxella,* the genus *Brevundimonas,* the genus *Enhydrobacter,* the genus *Comamonas,* and the genus *Rhodococcus;* OR
(iii) an asthma patient-derived sample wherein the extracellular vesicles are derived from one or more bacteria selected from the group consisting of:
the phylum *Bacteroidetes,* the phylum *Cyanobacteria,* the phylum TM7, the phylum *Fusobacteria,* the phylum *Thermi,* the phylum *Verrucomicrobia,* the phylum *Armatimonadetes,* the phylum *Acidobacteria,* the phylum *Gemmatimonadetes,* and the phylum *Chloroflexi;*
the class *Bacteroidia,* the class *Bacilli,* the class *Flavobacteriia,* the class *Sphingobacteriia,* the class *Alphaproteobacteria,* the class *Fusobacteriia,* the class TM7-3, the class *Deinococci,* the class *Verrucomicrobiae,* the class *Saprospirae,* the class *Chloroplast,* the class *Cytophagia,* the class *Fimbriimonadia,* the class *Chloracidobacteria,* the class *Thermomicrobia,* the class *Thermoleophilia,* and the class *Solibacteres;*
the order YS2, the order *Turicibacterales,* the order *Bifidobacteriales,* the order *Bacteroidales,* the order *Enterobacteriales,* the order *Rhodobacterales,* the order *Gemellales,* the order *Flavobacteriales,* the order *Caulobacterales,* the order *Neisseriales,* the order *Sphingobacteriales,* the order *Deinococcales,* the order *Pseudomonadales,* the order *Rhodocyclales,* the order *Xanthomonadales,* the order *Fusobacteriales,* the order *Actinomycetales,* the order *Sphingomonadales,* the order *Verrucomicrobiales,* the order *Saprospirales,* the order *Rhizobiales,* the order *Bacillales,* the order *Streptophyta,* the order *Cytophagales,* the order *Thermales,* the order *Fimbriimonadales,* the order CW040, the order *Rickettsiales,* the order RB41, the order *Alteromonadales,* the order JG30-KF-CM45, the order 1025, the order *Aeromonadales,* and the order *Solibacterales;*
the family *Helicobacteraceae,* the family *Bacteroidaceae,* the family *Turicibacteraceae,* the family *Veillonellaceae,* the family *Bifidobacteriaceae,* the family *Barnesiellaceae,* the family *Rikenellaceae,* the family *Clostridiaceae,* the family *Odoribacteraceae,* the family *Enterobacteriaceae,* the family *Porphyromonadaceae,* the family *Gemellaceae,* the family *Weeksellaceae,* the family *Carnobacteriaceae,* the family *Leptotrichiaceae,* the family *Moraxellaceae,* the family *Caulobacteraceae,* the family *Erythrobacteraceae,* the family *Hyphomicrobiaceae,* the family *Neisseriaceae,* the family *Sphingobacteriaceae,* the family *Deinococcaceae,* the family *Aerococcaceae,* the family *Bartonellaceae,* the family *Micrococcaceae,* the family *Flavobacteriaceae,* the family *Burkholderiaceae,* the family *Lactobacillaceae,* the family *Dietziaceae,* the family *Rhodocyclaceae,* the family *Xanthomonadaceae,* the family *Geodermatophilaceae,* the family *Actinomycetaceae,* the family *Methylobacteriaceae,* the family *Pseudomonadaceae,* the family *Corynebacteriaceae,* the family *Nocardioidaceae,* the family *Verrucomicrobiaceae,* the family *Sphingomonadaceae,* the family *Mycobacteriaceae,* the family *Tissierellaceae,* the family *Chitinophagaceae,* the family *Intrasporangiaceae,* the family *Propionibacteriaceae,* the family *Aurantimonadaceae,* the family *Planococcaceae,* the family *Fusobacteriaceae,* the family *Bradyrhizobiaceae,* the family *Nocardiaceae,* the family *Dermabacteraceae,* the family *Bacillaceae,* the family *Thermaceae,* the family Ellin6075, the family *Brevibacteriaceae,* the family *Microbacteriaceae,* the family *Rhodospirillaceae,* the family *Cytophagaceae,* the family *Fimbriimonadaceae,* the family *Dermacoccaceae,* the family *Chromatiaceae,* the family *Rhizobiaceae,* the family *Gordoniaceae,* the family *mitochondria,* the family *Pseudonocardiaceae,* the family *Exiguobacteraceae,* the family *Shewanellaceae,* the family F16, the family Rs-045, and the family *Aeromonadaceae;* and
the genus *Trabulsiella,* the genus *Veillonella,* the genus *Bifidobacterium,* the genus *Lachnospira,* the genus *Comamonas,* the genus *Bacteroides,* the genus *Turicibacter,* the genus *Sutterella,* the genus *Klebsiella,* the genus SMB53, the genus *Roseburia,* the genus *Dialister,* the genus *Ruminococcus,* the genus *Parabacteroides,* the genus *Butyricimonas,* the genus *Odoribacter,* the genus *Eubacterium,* the genus *Dorea,* the genus *Enhydrobacter,* the genus *Granulicatella,* the genus *Chryseobacterium,* the genus *Porphyromonas,* the genus *Coprococcus,* the genus *Peptoniphilus,* the genus *Microbispora,* the genus *Deinococcus,* the genus *Aerococcus,* the genus *Actinomyces,* the genus *Brevundimonas,* the genus *Blastomonas,* the genus *Citrobacter,* the genus *Lactobacillus,* the genus *Stenotrophomonas,* the genus *Corynebacterium,* the genus *Lautropia,* the genus *Akkermansia,* the genus *Bacillus,* the genus *Sphingobacterium,* the genus *Anaerococcus,* the genus *Neisseria,* the genus *Leptotrichia,* the genus *Mycobacterium,* the genus *Kocuria,* the genus *Methylobacterium,* the genus *Propionibacterium,* the genus *Hymenobacter,* the genus *Sphingomonas,* the genus *Fusobacterium,* the genus *Brachybacterium,* the genus *Rhodococcus,* the genus *Micrococcus,* the genus *Kaistobacter,* the genus *Finegoldia,* the genus *Rubellimicrobium,* the genus *Brevibacterium,* the genus *Agrobacterium,* the genus *Dietzia,* the genus *Fimbriimonas,* the genus *Flavobacterium,* the genus *Dermacoccus,* the genus *Skermanella,* the genus *Novosphingobium,* the genus *Gordonia,* the genus *Rheinheimera,* the genus *Achromobacter,* the genus *Hydrogenophilus,* the genus *Thermus,* the genus *Exiguobacterium,* the genus *Shewanella,* the genus *Ralstonia,* and the genus *Alkanindiges;*
and wherein the subject sample is blood.

3. The method of claim 1 or claim 2, wherein the blood is whole blood, serum, plasma, or blood mononuclear cells.

## Patentansprüche

1. Verfahren zum Bereitstellen von Informationen über eine Lungenkrebsdiagnose, wobei das Verfahren die folgenden Prozesse umfasst:
(a) Extrahieren von DNA aus extrazellulären Vesikeln, die aus einer Patientenprobe isoliert wurden;
(b) Durchführen einer Polymerase-Kettenreaktion (PCR) an der extrahierten DNA unter Verwendung eines Primer-Paares, das SEQ ID Nr: 1 und SEQ ID Nr: 2 aufweist; und
(c) Erstellen der Diagnose durch Vergleichen, durch Sequenzierung des PCR-Produkts, einer Erhöhung oder Verringerung eines Gehalts von bakterienabgeleiteten, extrazellulären Vesikeln der Patientenprobe mit jener
(i) einer von einem normalen Individuum stammenden Probe, wobei die extrazellulären Vesikel von einem oder mehreren Bakterien abgeleitet sind, ausgewählt aus der Gruppe bestehend aus:
dem Stamm Acidobacteria und dem Stamm Chloroflexi; der Klasse Thermomicrobia und der Klasse Solibacteres;
der Ordnung Turicibacterales, der Ordnung Rickettsiales, der Ordnung Alteromonadales, der Ordnung RF32, der Ordnung Thermales, der Ordnung JG30-KF-CM45, der Ordnung 1025, der Ordnung Solibacterales und der Ordnung Aeromonadales;
der Familie Turicibacteraceae, der Familie Clostridiaceae, der Familie S24-7, der Familie Rhizobiaceae, der Familie Mitochondrien, der Familie F16, der Familie Gordoniaceac, der Familie Rhodospirillaceae, der Familie Thermaceae, der Familie Shewanellaceae, der Familie Ellin6075, der Familie Rs-045 und der Familie Aeromonadaceae; und
der Gattung Chromohalobacter, der Gattung Geobacillus, der Gattung Proteus, der Gattung Megamonas, der Gattung Moraxella, der Gattung Alloiococcus, der Gattung Turicibacter, der Gattung SMB53, der Gattung Veillonella, der Gattung Peptoniphilus, der Gattung Comamonas, der Gattung Hymenobacter, der Gattung Citrobacter, der Gattung Novosphingobium, der Gattung Gordonia, der Gattung Aerococcus, der Gattung Thermus, der Gattung Shewanella und der Gattung Achromobacter;
(ii) einer von einem Patienten mit chronisch obstruktiver Lungenerkrankung stammenden Probe, wobei die extrazellulären Vesikel von einem oder mehreren Bakterien abgeleitet sind, ausgewählt aus der Gruppe bestehend aus:
der Ordnung Bacillales, der Ordnung Rickettsiales und der Ordnung 1025;
der Familie Nocardiaceae und der Familie Rs-045; und
der Gattung Alloiococcus, der Gattung Moraxella, der Gattung Brevundimonas, der Gattung Enhydrobacter, der Gattung Comamonas und der Gattung Rhodococcus; ODER
(iii) einer von einem Asthmapatienten stammenden Probe, wobei die extrazellulären Vesikel von einem oder mehreren Bakterien abgeleitet sind, ausgewählt aus der Gruppe bestehend aus:
dem Stamm Bacteroidetes, dem Stamm Cyanobacteria, dem Stamm TM7, dem Stamm Fusobacteria, dem Stamm Thermi, dem Stamm Verrucomicrobia, dem Stamm Armatimonadetes, dem Stamm Acidobacteria, dem Stamm Gemmatimonadetes und dem Stamm Chloroflexi;
der Klasse Bacteroidia, der Klasse Bacilli, der Klasse Flavobacteriia, der Klasse Sphingobacteriia, der Klasse Alphaproteobacteria, der Klasse Fusobacteriia, der Klasse TM7-3, der Klasse Deinococci, der Klasse Verrucomicrobiae, der Klasse Saprospirae, der Klasse Chloroplast, der Klasse Cytophagia, der Klasse Fimbriimonadia, der Klasse Chloracidobacteria, der Klasse Thermomicrobia, der Klasse Thermoleophilia und der Klasse Solibacteres;
der Ordnung YS2, der Ordnung Turicibacterales, der Ordnung Bifidobacteriales, der Ordnung Bacteroidales, der Ordnung Enterobacteriales, der Ordnung Rhodobacterales, der Ordnung Gemellales, der Ordnung Flavobacteriales, der Ordnung Caulobacterales, der Ordnung Neisseriales, der Ordnung Sphingobacteriales, der Ordnung Deinococcales, der Ordnung Pseudomonadales, der Ordnung Rhodocyclales, der Ordnung Xanthomonadales, der Ordnung Fusobacteriales, der Ordnung Actinomycetales, der Ordnung Sphingomonadales, der Ordnung Verrucomicrobiales, der Ordnung Saprospirales, der Ordnung Rhizobiales, der Ordnung Bacillales, der Ordnung Streptophyta, der Ordnung Cytophagales, der Ordnung Thermales, der Ordnung Fimbriimonadales, der Ordnung CW040, der Ordnung Rickettsiales, der Ordnung RB41, der Ordnung Alteromonadales, der Ordnung JG30-KF-CM45, der Ordnung 1025, der Ordnung Aeromonadales und der Ordnung Solibacterales;
der Familie Helicobacteraceae, der Familie Bacteroidaceae, der Familie Turicibacteraceae, der Familie Veillonellaceae, der Familie Bifidobacteriaceae, der Familie Barnesiellaceae, der Familie Rikenellaceae, der Familie Clostridiaceae, der Familie Odoribacteraceae, der Familie Enterobacteriaceae, der Familie Porphyromonadaceae, der Familie Gemellaceae, der Familie Weeksellaceae, der Familie Carnobacteriaceae, der Familie Leptotrichiaceae, der Familie Moraxellaceae, der Familie Caulobacteraceae, der Familie Erythrobacteraceae, der Familie Hyphomicrobiaceae, der Familie Neisseriaceae, der Familie Sphingobacteriaceae, der Familie Deinococcaceae, der Familie Aerococcaceae, der Familie Bartonellaceae, der Familie Micrococcaceae, der Familie Flavobacteriaceae, der Familie Burkholderiaceae, der Familie Lactobacillaceae, der Familie Dietziaceae, der Familie Rhodocyclaceae, der Familie Xanthomonadaceae, der Familie Geodermatophilaceae, der Familie Actinomycetaceae, der Familie Methylobacteriaceae, der Familie Pseudomonadaceae, der Familie Corynebacteriaceae, der Familie Nocardioidaceae, der Familie Verrucomicrobiaceae, der Familie Sphingomonadaceae, der Familie Mycobacteriaceae, der Familie Tissierellaceae, der Familie Chitinophagaceae, der Familie Intrasporangiaceae, der Familie Propionibacteriaceae, der Familie Aurantimonadaceae, der Familie Planococcaceae, der Familie Fusobacteriaceae, der Familie Bradyrhizobiaceae, der Familie Nocardiaceae, der Familie Dermabacteraceae, der Familie Bacillaceae, der Familie Thermaceae, der Familie Ellin6075, der Familie Brevibacteriaceae, der Familie Microbacteriaceae, der Familie Rhodospirillaceae, der Familie Cytophagaceae, der Familie Fimbriimonadaceae, der Familie Dermacoccaceae, der Familie Chromatiaceae, der Familie Rhizobiaceae, der Familie Gordoniaceae, der Familie mitochondria, der Familie Pseudonocardiaceae, der Familie Exiguobacteraceae, der Familie Shewanellaceae, der Familie F16, der Familie Rs-045 und der Familie Aeromonadaceae; und
der Gattung Trabulsiella, der Gattung Veillonella, der Gattung Bifidobacterium, der Gattung Lachnospira, der Gattung Comamonas, der Gattung Bacteroides, der Gattung Turicibacter, der Gattung Sutterella, der Gattung Klebsiella, der Gattung SMB53, der Gattung Roseburia, der Gattung Dialister, der Gattung Ruminococcus, der Gattung Parabacteroides, der Gattung Butyricimonas, der Gattung Odoribacter, der Gattung Eubacterium, der Gattung Dorea, der Gattung Enhydrobacter, der Gattung Granulicatella, der Gattung Chryseobacterium, der Gattung Porphyromonas, der Gattung Coprococcus, der Gattung Peptoniphilus, der Gattung Microbispora, der Gattung Deinococcus, der Gattung Aerococcus, der Gattung Actinomyces, der Gattung Brevundimonas, der Gattung Blastomonas, der Gattung Citrobacter, der Gattung Lactobacillus, der Gattung Stenotrophomonas, der Gattung Corynebacterium, der Gattung Lautropia, der Gattung Akkermansia, der Gattung Bacillus, der Gattung Sphingobacterium, der Gattung Anaerococcus, der Gattung Neisseria, der Gattung Leptotrichia, der Gattung Mycobacterium, der Gattung Kocuria, der Gattung Methylobacterium, der Gattung Propionibacterium, der Gattung Hymenobacter, der Gattung Sphingomonas, der Gattung Fusobacterium, der Gattung Brachybacterium, der Gattung Rhodococcus, der Gattung Micrococcus, der Gattung Kaistobacter, der Gattung Finegoldia, der Gattung Rubellimicrobium, der Gattung Brevibacterium, der Gattung Agrobacterium, der Gattung Dietzia, der Gattung Fimbriimonas, der Gattung Flavobacterium, der Gattung Dermacoccus, der Gattung Skermanella, der Gattung Novosphingobium, der Gattung Gordonia, der Gattung Rheinheimera, der Gattung Achromobacter, der Gattung Hydrogenophilus, der Gattung Thermus, der Gattung Exiguobacterium, der Gattung Shewanella, der Gattung Ralstonia und der Gattung Alkanindiges;
und wobei es sich bei der Patientenprobe um Blut handelt.

2. Verfahren zum Diagnostizieren von Lungenkrebs, wobei das Verfahren die folgenden Prozesse umfasst:
(a) Extrahieren von DNA aus extrazellulären Vesikeln, die aus einer Patientenprobe isoliert wurden;
(b) Durchführen einer Polymerase-Kettenreaktion (PCR) an der extrahierten DNA unter Verwendung eines Primer-Paares, das SEQ ID Nr: 1 und SEQ ID Nr: 2 aufweist; und
(c) Erstellen der Diagnose durch Vergleichen, durch Sequenzierung eines Produkts der PCR, einer Erhöhung oder Verringerung eines Gehalts von bakterienabgeleiteten, extrazellulären Vesikeln der Patientenprobe mit jener
(i) einer von einem normalen Individuum stammenden Probe, wobei die extrazellulären Vesikel von einem oder mehreren Bakterien abgeleitet sind, ausgewählt aus der Gruppe bestehend aus:
dem Stamm Acidobacteria und dem Stamm Chloroflexi; der Klasse Thermomicrobia und der Klasse Solibacteres;
der Ordnung Turicibacterales, der Ordnung Rickettsiales, der Ordnung Alteromonadales, der Ordnung RF32, der Ordnung Thermales, der Ordnung JG30-KF-CM45, der Ordnung 1025, der Ordnung Solibacterales und der Ordnung Aeromonadales;
der Familie Turicibacteraceae, der Familie Clostridiaceae, der Familie S24-7, der Familie Rhizobiaceae, der Familie Mitochondrien, der Familie F16, der Familie Gordoniaceae, der Familie Rhodospirillaceae, der Familie Thermaceae, der Familie Shewanellaceae, der Familie Ellin6075, der Familie Rs-045 und der Familie Aeromonadaceae; und
der Gattung Chromohalobacter, der Gattung Geobacillus, der Gattung Proteus, der Gattung Megamonas, der Gattung Moraxella, der Gattung Alloiococcus, der Gattung Turicibacter, der Gattung SMB53, der Gattung Veillonella, der Gattung Peptoniphilus, der Gattung Comamonas, der Gattung Hymenobacter, der Gattung Citrobacter, der Gattung Novosphingobium, der Gattung Gordonia, der Gattung Aerococcus, der Gattung Thermus, der Gattung Shewanella und der Gattung Achromobacter;
(ii) einer von einem Patienten mit chronisch obstruktiver Lungenerkrankung stammenden Probe, wobei die extrazellulären Vesikel von einem oder mehreren Bakterien abgeleitet sind, ausgewählt aus der Gruppe bestehend aus:
der Ordnung Bacillales, der Ordnung Rickettsiales und der Ordnung 1025;
der Familie Nocardiaceae und der Familie Rs-045; und
der Gattung Alloiococcus, der Gattung Moraxella, der Gattung Brevundimonas, der Gattung Enhydrobacter, der Gattung Comamonas und der Gattung Rhodococcus; ODER
(iii) einer von einem Asthmapatienten stammenden Probe, wobei die extrazellulären Vesikel von einem oder mehreren Bakterien abgeleitet sind, ausgewählt aus der Gruppe bestehend aus:
dem Stamm Bacteroidetes, dem Stamm Cyanobacteria, dem Stamm TM7, dem Stamm Fusobacteria, dem Stamm Thermi, dem Stamm Verrucomicrobia, dem Stamm Armatimonadetes, dem Stamm Acidobacteria, dem Stamm Gemmatimonadetes und dem Stamm Chloroflexi;
der Klasse Bacteroidia, der Klasse Bacilli, der Klasse Flavobacteriia, der Klasse Sphingobacteriia, der Klasse Alphaproteobacteria, der Klasse Fusobacteriia, der Klasse TM7-3, der Klasse Deinococci, der Klasse Verrucomicrobiae, der Klasse Saprospirae, der Klasse Chloroplast, der Klasse Cytophagia, der Klasse Fimbriimonadia, der Klasse Chloracidobacteria, der Klasse Thermomicrobia, der Klasse Thermoleophilia und der Klasse Solibacteres;
der Ordnung YS2, der Ordnung Turicibacterales, der Ordnung Bifidobacteriales, der Ordnung Bacteroidales, der Ordnung Enterobacteriales, der Ordnung Rhodobacterales, der Ordnung Gemellales, der Ordnung Flavobacteriales, der Ordnung Caulobacterales, der Ordnung Neisseriales, der Ordnung Sphingobacteriales, der Ordnung Deinococcales, der Ordnung Pseudomonadales, der Ordnung Rhodocyclales, der Ordnung Xanthomonadales, der Ordnung Fusobacteriales, der Ordnung Actinomycetales, der Ordnung Sphingomonadales, der Ordnung Verrucomicrobiales, der Ordnung Saprospirales, der Ordnung Rhizobiales, der Ordnung Bacillales, der Ordnung Streptophyta, der Ordnung Cytophagales, der Ordnung Thermales, der Ordnung Fimbriimonadales, der Ordnung CW040, der Ordnung Rickettsiales, der Ordnung RB41, der Ordnung Alteromonadales, der Ordnung JG30-KF-CM45, der Ordnung 1025, der Ordnung Aeromonadales und der Ordnung Solibacterales;
der Familie Helicobacteraceae, der Familie Bacteroidaceae, der Familie Turicibacteraceae, der Familie Veillonellaceae, der Familie Bifidobacteriaceae, der Familie Barnesiellaceae, der Familie Rikenellaceae, der Familie Clostridiaceae, der Familie Odoribacteraceae, der Familie Enterobacteriaceae, der Familie Porphyromonadaceae, der Familie Gemellaceae, der Familie Weeksellaceae, der Familie Carnobacteriaceae, der Familie Leptotrichiaceae, der Familie Moraxellaceae, der Familie Caulobacteraceae, der Familie Erythrobacteraceae, der Familie Hyphomicrobiaceae, der Familie Neisseriaceae, der Familie Sphingobacteriaceae, der Familie Deinococcaceae, der Familie Aerococcaceae, der Familie Bartonellaceae, der Familie Micrococcaceae, der Familie Flavobacteriaceae, der Familie Burkholderiaceae, der Familie Lactobacillaceae, der Familie Dietziaceae, der Familie Rhodocyclaceae, der Familie Xanthomonadaceae, der Familie Geodermatophilaceae, der Familie Actinomycetaceae, der Familie Methylobacteriaceae, der Familie Pseudomonadaceae, der Familie Corynebacteriaceae, der Familie Nocardioidaceae, der Familie Verrucomicrobiaceae, der Familie Sphingomonadaceae, der Familie Mycobacteriaceae, der Familie Tissierellaceae, der Familie Chitinophagaceae, der Familie Intrasporangiaceae, der Familie Propionibacteriaceae, der Familie Aurantimonadaceae, der Familie Planococcaceae, der Familie Fusobacteriaceae, der Familie Bradyrhizobiaceae, der Familie Nocardiaceae, der Familie Dermabacteraceae, der Familie Bacillaceae, der Familie Thermaceae, der Familie Ellin6075, der Familie Brevibacteriaceae, der Familie Microbacteriaceae, der Familie Rhodospirillaceae, der Familie Cytophagaceae, der Familie Fimbriimonadaceae, der Familie Dermacoccaceae, der Familie Chromatiaceae, der Familie Rhizobiaceae, der Familie Gordoniaceae, der Familie mitochondria, der Familie Pseudonocardiaceae, der Familie Exiguobacteraceae, der Familie Shewanellaceae, der Familie F16, der Familie Rs-045 und der Familie Aeromonadaceae; und
der Gattung Trabulsiella, der Gattung Veillonella, der Gattung Bifidobacterium, der Gattung Lachnospira, der Gattung Comamonas, der Gattung Bacteroides, der Gattung Turicibacter, der Gattung Sutterella, der Gattung Klebsiella, der Gattung SMB53, der Gattung Roseburia, der Gattung Dialister, der Gattung Ruminococcus, der Gattung Parabacteroides, der Gattung Butyricimonas, der Gattung Odoribacter, der Gattung Eubacterium, der Gattung Dorea, der Gattung Enhydrobacter, der Gattung Granulicatella, der Gattung Chryseobacterium, der Gattung Porphyromonas, der Gattung Coprococcus, der Gattung Peptoniphilus, der Gattung Microbispora, der Gattung Deinococcus, der Gattung Aerococcus, der Gattung Actinomyces, der Gattung Brevundimonas, der Gattung Blastomonas, der Gattung Citrobacter, der Gattung Lactobacillus, der Gattung Stenotrophomonas, der Gattung Corynebacterium, der Gattung Lautropia, der Gattung Akkermansia, der Gattung Bacillus, der Gattung Sphingobacterium, der Gattung Anaerococcus, der Gattung Neisseria, der Gattung Leptotrichia, der Gattung Mycobacterium, der Gattung Kocuria, der Gattung Methylobacterium, der Gattung Propionibacterium, der Gattung Hymenobacter, der Gattung Sphingomonas, der Gattung Fusobacterium, der Gattung Brachybacterium, der Gattung Rhodococcus, der Gattung Micrococcus, der Gattung Kaistobacter, der Gattung Finegoldia, der Gattung Rubellimicrobium, der Gattung Brevibacterium, der Gattung Agrobacterium, der Gattung Dietzia, der Gattung Fimbriimonas, der Gattung Flavobacterium, der Gattung Dermacoccus, der Gattung Skermanella, der Gattung Novosphingobium, der Gattung Gordonia, der Gattung Rheinheimera, der Gattung Achromobacter, der Gattung Hydrogenophilus, der Gattung Thermus, der Gattung Exiguobacterium, der Gattung Shewanella, der Gattung Ralstonia und der Gattung Alkanindiges;
und wobei es sich bei der Patientenprobe um Blut handelt.

3. Verfahren nach Anspruch 1 oder Anspruch 2, wobei das Blut Vollblut, Serum, Plasma oder mononukleäre Blutzellen ist.

## Revendications

1. Procédé de fourniture d'informations pour le diagnostic du cancer du poumon, le procédé comprenant les processus suivants :
(a) l'extraction d'ADN de vésicules extracellulaires isolées à partir d'un échantillon de sujet ;
(b) la réalisation d'une réaction en chaîne par polymérase (PCR) sur l'ADN extrait à l'aide d'une paire d'amorces présentant SEQ ID NO : 1 et SEQ ID NO : 2 ; et
(c) l'établissement dudit diagnostic, par l'intermédiaire du séquençage du produit PCR, en comparant une augmentation ou une diminution de la teneur en vésicules extracellulaires dérivées de bactéries de l'échantillon de sujet à celle de
(i) un échantillon dérivé d'un individu normal, dans lequel les vésicules extracellulaires sont dérivées d'une ou plusieurs bactéries sélectionnées dans le groupe consistant en :
le phylum Acidobacteria et le phylum Chloroflexi ; la classe Thermomicrobia et la classe Solibacteres ;
l'ordre Turicibactérales, l'ordre Rickettsiales, l'ordre Altéromonadales, l'ordre RF32, l'ordre Thermales, l'ordre JG30-KF-CM45, l'ordre 1025, l'ordre Solibactérales et l'ordre Aéromonadales ;
la famille Turicibacteraceae, la famille Clostridiaceae, la famille S24-7, la famille Rhizobiaceae, la famille mitochondries, la famille F16, la famille Gordoniaceae, la famille Rhodospirillaceae, la famille Thermaceae, la famille Shewanellaceae, la famille Ellin6075, la famille Rs-045, et la famille Aeromonadaceae ; et
le genre Chromohalobacter, le genre Geobacillus, le genre Proteus, le genre Megamonas, le genre Moraxella, le genre Alloiococcus, le genre Turicibacter, le genre SMB53, le genre Veillonella, le genre Peptoniphilus, le genre Comamonas, le genre Hymenobacter, le genre Citrobacter, le genre Novosphingobium, le genre Gordonia, le genre Aerococcus, le genre Thermus, le genre Shewanella et le genre Achromobacter ;
(ii) un échantillon dérivé d'un patient atteint d'une maladie pulmonaire obstructive chronique, dans lequel les vésicules extracellulaires sont dérivées d'une ou plusieurs bactéries sélectionnées dans le groupe consistant en :
l'ordre Bacillales, l'ordre Rickettsiales et l'ordre 1025 ;
la famille Nocardiaceae et la famille Rs-045 ; et
le genre Alloiococcus, le genre Moraxella, le genre Brevundimonas, le genre Enhydrobacter, le genre Comamonas et le genre Rhodococcus ; OU
(iii) un échantillon dérivé d'un patient asthmatique, dans lequel les vésicules extracellulaires sont dérivées d'une ou plusieurs bactéries sélectionnées dans le groupe consistant en :
le phylum Bacteroidetes, le phylum Cyanobacteria, le phylum TM7, le phylum Fusobacteria, le phylum Thermi, le phylum Verrucomicrobia, le phylum Armatimonadetes, le phylum Acidobacteria, le phylum Gemmatimonadetes et le phylum Chloroflexi ;
la classe Bacteroidia, la classe Bacilli, la classe Flavobacteriia, la classe Sphingobacteriia, la classe Alphaproteobacteria, la classe Fusobacteriia, la classe TM7-3, la classe Deinococci, la classe Verrucomicrobiae, la classe Saprospirae, la classe Chloroplaste, la classe Cytophagia, la classe Fimbriimonadia, la classe Chloracidobacteria, la classe Thermomicrobia, la classe Thermoleophilia et la classe Solibacteres ;
l'ordre YS2, l'ordre Turicibactérales, l'ordre Bifidobactériales, l'ordre Bactéroïdales, l'ordre Enterobactériales, l'ordre Rhodobactérales, l'ordre Gemellales, l'ordre Flavobactériales, l'ordre Caulobactérales, l'ordre Neissériales, l'ordre Sphingobactériales, l'ordre Déinococcales, l'ordre Pseudomonadales, l'ordre Rhodocyclales, l'ordre Xanthomonadales, l'ordre Fusobactériales, l'ordre Actinomycétales, l'ordre Sphingomonadales, l'ordre Verrucomicrobiales, l'ordre Saprospirales, l'ordre Rhizobiales, l'ordre Bacillales, l'ordre Streptophyta, l'ordre Cytophagales, l'ordre Thermales, l'ordre Fimbriimonadales, l'ordre CW040, l'ordre Rickettsiales, l'ordre RB41, l'ordre Altéromonadales, l'ordre JG30-KF-CM45, l'ordre 1025, l'ordre Aéromonadales, et l'ordre Solibactérales ;
la famille Helicobacteraceae, la famille Bacteroidaceae, la famille Turicibacteraceae, la famille Veillonellaceae, la famille Bifidobacteriaceae, la famille Barnesiellaceae, la famille Rikenellaceae, la famille Clostridiaceae, la famille Odoribacteraceae, la famille Enterobacteriaceae, la famille Porphyromonadaceae, la famille Gemellaceae, la famille Weeksellaceae, la famille Carnobacteriaceae, la famille Leptotrichiaceae, la famille Moraxellaceae, la famille Caulobacteraceae, la famille Erythrobacteraceae, la famille Hyphomicrobiaceae, la famille Neisseriaceae, la famille Sphingobacteriaceae, la famille Deinococcaceae, la famille Aerococcaceae, la famille Bartonellaceae, la famille Micrococcaceae, la famille Flavobacteriaceae, la famille Burkholderiaceae, la famille Lactobacillaceae, la famille Dietziaceae, la famille Rhodocyclaceae, la famille Xanthomonadaceae, la famille Geodermatophilaceae, la famille Actinomycetaceae, la famille Methylobacteriaceae, la famille Pseudomonadaceae, la famille Corynebacteriaceae, la famille Nocardioidaceae, la famille Verrucomicrobiaceae, la famille Sphingomonadaceae, la famille Mycobacteriaceae, la famille Tissierellaceae, la famille Chitinophagaceae, la famille Intrasporangiaceae, la famille Propionibacteriaceae, la famille Aurantimonadaceae, la famille Planococcaceae, la famille Fusobacteriaceae, la famille Bradyrhizobiaceae, la famille Nocardiaceae, la famille Dermabacteraceae, la famille Bacillaceae, la famille Thermaceae, la famille Ellin6075, la famille Brevibacteriaceae, la famille Microbacteriaceae, la famille Rhodospirillaceae, la famille Cytophagaceae, la famille Fimbriimonadaceae, la famille Dermacoccaceae, la famille Chromatiaceae, la famille Rhizobiaceae, la famille Gordoniaceae, la famille mitochondria, la famille Pseudonocardiaceae, la famille Exiguobacteraceae, la famille Shewanellaceae, la famille F16, la famille Rs-045, et la famille Aeromonadaceae ; et
le genre Trabulsiella, le genre Veillonella, le genre Bifidobacterium, le genre Lachnospira, le genre Comamonas, le genre Bacteroides, le genre Turicibacter, le genre Sutterella, le genre Klebsiella, le genre SMB53, le genre Roseburia, le genre Dialister, le genre Ruminococcus, le genre Parabacteroides, le genre Butyricimonas, le genre Odoribacter, le genre Eubacterium, le genre Dorea, le genre Enhydrobacter, le genre Granulicatella, le genre Chryseobacterium, le genre Porphyromonas, le genre Coprococcus, le genre Peptoniphilus, le genre Microbispora, le genre Deinococcus, le genre Aerococcus, le genre Actinomyces, le genre Brevundimonas, le genre Blastomonas, le genre Citrobacter, le genre Lactobacillus, le genre Stenotrophomonas, le genre Corynebacterium, le genre Lautropia, le genre Akkermansia, le genre Bacillus, le genre Sphingobacterium, le genre Anaerococcus, le genre Neisseria, le genre Leptotrichia, le genre Mycobacterium, le genre Kocuria, le genre Methylobacterium, le genre Propionibacterium, le genre Hymenobacter, le genre Sphingomonas, le genre Fusobacterium, le genre Brachybacterium, le genre Rhodococcus, le genre Micrococcus, le genre Kaistobacter, le genre Finegoldia, le genre Rubellimicrobium, le genre Brevibacterium, le genre Agrobacterium, le genre Dietzia, le genre Fimbriimonas, le genre Flavobacterium, le genre Dermacoccus, le genre Skermanella, le genre Novosphingobium, le genre Gordonia, le genre Rheinheimera, le genre Achromobacter, le genre Hydrogenophilus, le genre Thermus, le genre Exiguobacterium, le genre Shewanella, le genre Ralstonia, et le genre Alkanindiges ;
et dans lequel l'échantillon de sujet est du sang.

2. Procédé de diagnostic du cancer du poumon, le procédé comprenant les processus suivants :
(a) l'extraction d'ADN de vésicules extracellulaires isolées à partir d'un échantillon de sujet ;
(b) la réalisation d'une réaction en chaîne par polymérase (PCR) sur l'ADN extrait à l'aide d'une paire d'amorces présentant SEQ ID NO : 1 et SEQ ID NO : 2 ; et
(c) l'établissement dudit diagnostic, par l'intermédiaire du séquençage d'un produit de la PCR, en comparant une augmentation ou une diminution de la teneur en vésicules extracellulaires dérivées de bactéries de l'échantillon de sujet à celle de
(i) un échantillon dérivé d'un individu normal, dans lequel les vésicules extracellulaires sont dérivées d'une ou plusieurs bactéries sélectionnées dans le groupe consistant en :
le phylum Acidobacteria et le phylum Chloroflexi ; la classe Thermomicrobia et la classe Solibacteres ;
l'ordre Turicibactérales, l'ordre Rickettsiales, l'ordre Altéromonadales, l'ordre RF32, l'ordre Thermales, l'ordre JG30-KF-CM45, l'ordre 1025, l'ordre Solibactérales et l'ordre Aéromonadales ;
la famille Turicibacteraceae, la famille Clostridiaceae, la famille S24-7, la famille Rhizobiaceae, la famille mitochondries, la famille F16, la famille Gordoniaceae, la famille Rhodospirillaceae, la famille Thermaceae, la famille Shewanellaceae, la famille Ellin6075, la famille Rs-045, et la famille Aeromonadaceae ; et
le genre Chromohalobacter, le genre Geobacillus, le genre Proteus, le genre Megamonas, le genre Moraxella, le genre Alloiococcus, le genre Turicibacter, le genre SMB53, le genre Veillonella, le genre Peptoniphilus, le genre Comamonas, le genre Hymenobacter, le genre Citrobacter, le genre Novosphingobium, le genre Gordonia, le genre Aerococcus, le genre Thermus, le genre Shewanella et le genre Achromobacter ;
(ii) un échantillon dérivé d'un patient atteint d'une maladie pulmonaire obstructive chronique, dans lequel les vésicules extracellulaires sont dérivées d'une ou plusieurs bactéries sélectionnées dans le groupe consistant en :
l'ordre Bacillales, l'ordre Rickettsiales et l'ordre 1025 ;
la famille Nocardiaceae et la famille Rs-045 ; et
le genre Alloiococcus, le genre Moraxella, le genre Brevundimonas, le genre Enhydrobacter, le genre Comamonas et le genre Rhodococcus ; OU
(iii) un échantillon dérivé d'un patient asthmatique, dans lequel les vésicules extracellulaires sont dérivées d'une ou plusieurs bactéries sélectionnées dans le groupe consistant en :
le phylum Bacteroidetes, le phylum Cyanobacteria, le phylum TM7, le phylum Fusobacteria, le phylum Thermi, le phylum Verrucomicrobia, le phylum Armatimonadetes, le phylum Acidobacteria, le phylum Gemmatimonadetes et le phylum Chloroflexi ;
la classe Bacteroidia, la classe Bacilli, la classe Flavobacteriia, la classe Sphingobacteriia, la classe Alphaproteobacteria, la classe Fusobacteriia, la classe TM7-3, la classe Deinococci, la classe Verrucomicrobiae, la classe Saprospirae, la classe Chloroplaste, la classe Cytophagia, la classe Fimbriimonadia, la classe Chloracidobacteria, la classe Thermomicrobia, la classe Thermoleophilia et la classe Solibacteres ;
l'ordre YS2, l'ordre Turicibactérales, l'ordre Bifidobactériales, l'ordre Bactéroïdales, l'ordre Enterobactériales, l'ordre Rhodobactérales, l'ordre Gemellales, l'ordre Flavobactériales, l'ordre Caulobactérales, l'ordre Neissériales, l'ordre Sphingobactériales, l'ordre Déinococcales, l'ordre Pseudomonadales, l'ordre Rhodocyclales, l'ordre Xanthomonadales, l'ordre Fusobactériales, l'ordre Actinomycétales, l'ordre Sphingomonadales, l'ordre Verrucomicrobiales, l'ordre Saprospirales, l'ordre Rhizobiales, l'ordre Bacillales, l'ordre Streptophyta, l'ordre Cytophagales, l'ordre Thermales, l'ordre Fimbriimonadales, l'ordre CW040, l'ordre Rickettsiales, l'ordre RB41, l'ordre Altéromonadales, l'ordre JG30-KF-CM45, l'ordre 1025, l'ordre Aéromonadales, et l'ordre Solibactérales ;
la famille Helicobacteraceae, la famille Bacteroidaceae, la famille Turicibacteraceae, la famille Veillonellaceae, la famille Bifidobacteriaceae, la famille Barnesiellaceae, la famille Rikenellaceae, la famille Clostridiaceae, la famille Odoribacteraceae, la famille Enterobacteriaceae, la famille Porphyromonadaceae, la famille Gemellaceae, la famille Weeksellaceae, la famille Carnobacteriaceae, la famille Leptotrichiaceae, la famille Moraxellaceae, la famille Caulobacteraceae, la famille Erythrobacteraceae, la famille Hyphomicrobiaceae, la famille Neisseriaceae, la famille Sphingobacteriaceae, la famille Deinococcaceae, la famille Aerococcaceae, la famille Bartonellaceae, la famille Micrococcaceae, la famille Flavobacteriaceae, la famille Burkholderiaceae, la famille Lactobacillaceae, la famille Dietziaceae, la famille Rhodocyclaceae, la famille Xanthomonadaceae, la famille Geodermatophilaceae, la famille Actinomycetaceae, la famille Methylobacteriaceae, la famille Pseudomonadaceae, la famille Corynebacteriaceae, la famille Nocardioidaceae, la famille Verrucomicrobiaceae, la famille Sphingomonadaceae, la famille Mycobacteriaceae, la famille Tissierellaceae, la famille Chitinophagaceae, la famille Intrasporangiaceae, la famille Propionibacteriaceae, la famille Aurantimonadaceae, la famille Planococcaceae, la famille Fusobacteriaceae, la famille Bradyrhizobiaceae, la famille Nocardiaceae, la famille Dermabacteraceae, la famille Bacillaceae, la famille Thermaceae, la famille Ellin6075, la famille Brevibacteriaceae, la famille Microbacteriaceae, la famille Rhodospirillaceae, la famille Cytophagaceae, la famille Fimbriimonadaceae, la famille Dermacoccaceae, la famille Chromatiaceae, la famille Rhizobiaceae, la famille Gordoniaceae, la famille mitochondria, la famille Pseudonocardiaceae, la famille Exiguobacteraceae, la famille Shewanellaceae, la famille F16, la famille Rs-045, et la famille Aeromonadaceae ; et
le genre Trabulsiella, le genre Veillonella, le genre Bifidobacterium, le genre Lachnospira, le genre Comamonas, le genre Bacteroides, le genre Turicibacter, le genre Sutterella, le genre Klebsiella, le genre SMB53, le genre Roseburia, le genre Dialister, le genre Ruminococcus, le genre Parabacteroides, le genre Butyricimonas, le genre Odoribacter, le genre Eubacterium, le genre Dorea, le genre Enhydrobacter, le genre Granulicatella, le genre Chryseobacterium, le genre Porphyromonas, le genre Coprococcus, le genre Peptoniphilus, le genre Microbispora, le genre Deinococcus, le genre Aerococcus, le genre Actinomyces, le genre Brevundimonas, le genre Blastomonas, le genre Citrobacter, le genre Lactobacillus, le genre Stenotrophomonas, le genre Corynebacterium, le genre Lautropia, le genre Akkermansia, le genre Bacillus, le genre Sphingobacterium, le genre Anaerococcus, le genre Neisseria, le genre Leptotrichia, le genre Mycobacterium, le genre Kocuria, le genre Methylobacterium, le genre Propionibacterium, le genre Hymenobacter, le genre Sphingomonas, le genre Fusobacterium, le genre Brachybacterium, le genre Rhodococcus, le genre Micrococcus, le genre Kaistobacter, le genre Finegoldia, le genre Rubellimicrobium, le genre Brevibacterium, le genre Agrobacterium, le genre Dietzia, le genre Fimbriimonas, le genre Flavobacterium, le genre Dermacoccus, le genre Skermanella, le genre Novosphingobium, le genre Gordonia, le genre Rheinheimera, le genre Achromobacter, le genre Hydrogenophilus, le genre Thermus, le genre Exiguobacterium, le genre Shewanella, le genre Ralstonia, et le genre Alkanindiges ;
et dans lequel l'échantillon de sujet est du sang.

3. Procédé selon la revendication 1 ou la revendication 2, dans lequel le sang est du sang total, du sérum, du plasma ou des cellules mononucléaires sanguines.
